# EUROPEAN PATENT APPLICATION

(11) **EP 4 080 584 A1**
(43) Date of publication of application: **26.10.2022**
(21) Application number: 20902381.1
(22) Date of filing: 18.12.2020
(51) Int. Cl.: H01L 29/786, G01N 33/483, G01N 33/53, G01N 33/552, G01N 27/414

(54) **TRANSISTOR SENSOR, AND METHOD FOR DETECTING BIOMATERIALS**

(30) Priority: 20.12.2019 JP 2019230511; 17.12.2020 JP 2020209541
(71) Applicant: Mitsubishi Materials Corporation, Tokyo 100-8117 (JP); Japan Advanced Institute of Science and Technology, Ishikawa 923-1292 (JP)
(72) Inventor: NAKAZAWA Hiromi, Naka-shi, Ibaraki 311-0102 (JP); SOYAMA Nobuyuki, Naka-shi, Ibaraki 311-0102 (JP); SHIRATA Keiji, Naka-shi, Ibaraki 311-0102 (JP); DOI Toshihiro, Naka-shi, Ibaraki 311-0102 (JP); PHAN Tue Trong, Nomi-shi, Ishikawa 923-1292 (JP); TAKAMURA Yuzuru, Nomi-shi, Ishikawa 923-1292 (JP); SHIMODA Tatsuya, Suwa-gun, Nagano 399-0214 (JP); HIROSE Daisuke, Nomi-shi, Ishikawa 923-1292 (JP)
(74) Representative: Gille Hrabal Partnerschaftsgesellschaft mbB Patentanwälte
(86) International application number: PCT/JP2020/047485
(87) International publication number: WO 2021/125335

(57) **Abstract**

This transistor sensor includes a substrate, a channel layer provided over one surface of the substrate, and a solid electrolyte layer provided between the substrate and the channel layer or over a surface of the channel layer on an opposite side to the substrate side, in which the channel layer includes an inorganic semiconductor, the solid electrolyte layer includes an inorganic solid electrolyte, and at least a portion of either one or both of the channel layer and the solid electrolyte layer includes an exposed portion exposed to outside.

## Description

### TECHNICAL FIELD

The present invention relates to a transistor sensor and a method for detecting a biomaterial.

Priority is claimed on Japanese Patent Application No. 2019-230511 filed in Japan on December 20, 2019 and Japanese Patent Application No. 2020-209541 filed in Japan on December 17, 2020, the contents of which are hereby incorporated herein.

### BACKGROUND ART

DNA sensors using transistor sensors, such as thin film transistor (TFT) sensors, are widely known and various DNA sensors using transistor sensors have been reported up to now. For example, a method was reported in which a homo-oligomeric DNA strand is fixed using 3-aminopropyl ethoxysilane and hybridization with the above oligomeric strand is directly detected by the displacement of a gate potential under a constant drain current (Non-Patent Document 1).

In addition, as methods of the related art for measuring mRNA/DNA, there is the PCR method (polymerase chain reaction) and a method using next generation sequencing. The PCR method is a method in which a specific region (target region) on a DNA sequence is amplified using a heat-resistant DNA polymerase and, since it is possible to detect one DNA molecule, it is possible to detect a specific sequence of DNA with high sensitivity. In addition, next-generation sequencing is a method in which a library is prepared by fragmenting DNA and the DNA fragments of the library are sequenced in parallel and with which it is possible to comprehensively decipher an entire DNA sequence from one molecule.

### Citation List

### Non-Patent Document

[Non-Patent Document 1] J. Phys. Chem. B, 1997, 101, 2980-2985.

### SUMMARY OF INVENTION

### Technical Problem

However, in the technology of Non-Patent Document 1, the detection limit is not that high at approximately 1 µg/mL and the detection values change depending on the substances coexisting in the actual samples, thus, the detection limit and selectivity are insufficient. In addition, DNA sensors using the transistor sensors of the related art are easily influenced by moisture and salt and detection in the presence of moisture and salt is unstable.

In order to increase the detection limit, high sensitivity or low noise is necessary. The sensitivity of transistor sensors is determined by (mobility) x (gate capacitance) and the S-factor; however, the sensitivity of the transistor sensors of the related art is not that high. Thus, in order to increase the detection limit, detection stability at low concentrations is important; however, the transistor sensors of the related art have low stability in aqueous solutions, in particular, in real biological sample solutions in which proteins and the like coexist.

On the other hand, although the PCR method described above is highly sensitive and able to detect one DNA molecule, an amplification process is necessary, which takes time for the detection and is labor-intensive and costly. In addition, in the next generation sequencing described above, the detection time and cost are even higher. In recent years, the need for clinical testing in the medical field has been increasing year by year and there is a particular demand for rapid and highly sensitive detection and measurement in the analysis of infectious diseases, novel cancer markers, individual genes, exosomes, mRNA in leukocytes, cell-free RNA/DNA, and the like.

The object of the present invention is to provide a transistor sensor and a method for detecting a biomaterial, which have high sensitivity, a low detection limit value, and high detection stability and which are able to detect biomaterials rapidly and easily at low cost.

### Solution to Problem

As a result of intensive research, the present inventors found that, in a transistor sensor having a substrate, a channel layer provided over one surface of the substrate, and a gate insulating layer (solid electrolyte layer) provided between the substrate and the channel layer or over a surface of the channel layer on an opposite side to the substrate side, in which at least a portion of either one or both of the channel layer and the solid electrolyte layer is provided with an exposed portion exposed to the outside, by the channel layer including an inorganic semiconductor and the solid electrolyte layer including an inorganic solid electrolyte, it is possible to carry out high-sensitivity detection of biomaterials, the detection limit value is low, the detection stability is improved, and it is possible to rapidly and easily detect biomaterials at low cost. In addition, it was found that, by using a composite metal oxide including a specific plurality of metal elements as the gate insulating layer (solid electrolyte layer) of the transistor sensor, using a metal oxide (inorganic semiconductor) including one or a plurality of specific metal elements as the channel layer and, furthermore, setting the content of carbon and hydrogen included in the gate insulating layer (solid electrolyte layer) within a predetermined range, the detection limit value is much lower with higher sensitivity than in the transistor sensors of the related art, the stability in the presence of moisture and the like is higher, and it is possible to reduce the detection time, labor, and cost compared to the related art, thereby completing the present invention.

That is, the present invention provides the following means to solve the problems described above.
[1] A transistor sensor including a substrate, a channel layer provided over one surface of the substrate, and a solid electrolyte layer provided between the substrate and the channel layer or over a surface of the channel layer on an opposite side to the substrate side, in which the channel layer includes an inorganic semiconductor, the solid electrolyte layer includes an inorganic solid electrolyte, and at least a portion of either one or both of the channel layer and the solid electrolyte layer includes an exposed portion exposed to outside.
[2] The transistor sensor according to [1], in which the solid electrolyte layer is formed of any of a metal oxide including rare earth elements and zirconium (Zr) and a metal oxide including rare earth elements and tantalum (Ta), the channel layer is formed of a metal oxide including at least indium (In), a carbon (C) content ratio in the solid electrolyte layer is 0.5 atom% or more and 15 atom% or less, and a hydrogen (H) content ratio in the solid electrolyte layer is 2 atom% or more and 20 atom% or less.
[3] The transistor sensor according to [1] or [2], in which the solid electrolyte layer has an ionic conductivity of 1 x 10⁻⁸ S/cm or higher.
[4] The transistor sensor according to any one of [1] to [3], in which a surface of the exposed portion has a capturing field configured to capture a biomaterial directly or indirectly.
[5] The transistor sensor according to [4], in which a probe molecule for capturing the biomaterial is fixed to the capturing field.
[6] The transistor sensor according to any one of [1] to [5], further including a holding portion configured to hold a liquid including a biomaterial, in an upper portion of the exposed portion.
[7] The transistor sensor according to any one of [1] to [6], further including a conductive material layer in contact with at least a portion of the solid electrolyte layer, in which the conductive material layer is electrically insulated except at the solid electrolyte layer.
[8] The transistor sensor according to any one of [1] to [7], further including a source electrode and a drain electrode connected to the channel layer, a reference electrode which, when a liquid including a biomaterial is arranged in contact with the exposed portion, is inserted into the liquid including the biomaterial, and a detection portion configured to detect the biomaterial based on a voltage between the reference electrode and the source electrode and a current between the source electrode and the drain electrode.
[9] A method for detecting a biomaterial using the transistor sensor according to [8], the method including a step of supplying a liquid including a biomaterial to the exposed portion, a step of inserting the reference electrode into the liquid including the biomaterial, and a step of applying a voltage between the reference electrode and the source electrode of the transistor sensor, measuring a current between the source electrode and the drain electrode of the transistor sensor, and detecting the biomaterial based on the voltage between the reference electrode and the source electrode and the current between the source electrode and the drain electrode.

### Advantageous Effects of Invention

According to the present invention, it is possible to rapidly and easily detect biomaterials at low cost and to realize high detection stability with high sensitivity and a low detection limit value.

### BRIEF DESCRIPTION OF DRAWINGS

Fig. 1(a) is a cross-sectional view (cross-sectional view along line I-I of Fig. 1(b)) showing an example of a configuration of a transistor sensor according to a first embodiment of the present invention and Fig. 1(b) is a plan view thereof.
Fig. 2(a) to Fig. 2(d) are cross-sectional views showing an example of steps of a method for manufacturing the transistor sensor of Fig. 1(a).
Fig. 3 is a schematic diagram showing a method for detecting DNA using the transistor sensor of Fig. 1(a).
Fig. 4 is a graph showing V_{TG}-I_{DS} characteristics of the transistor sensor of Invention Example 1.
Fig. 5 is a graph showing V_{TG}-I_{DS} characteristics of a transistor sensor of the related art.
Fig. 6 is a cross-sectional view of a transistor sensor showing the action of a gate insulating layer (solid electrolyte layer) and a back gate electrode (conductive material layer).
Fig. 7(a) to Fig. 7(d) are schematic diagrams showing an example of a detection method in which the transistor sensor of Figs. 1 is used to repeatedly detect target DNA.
Fig. 8(a) and Fig. 8(b) are graphs showing a relationship between a top gate voltage V_{TG} and a drain current I_{DS} when the width of the channel layer is changed in the transistor sensor of Fig. 1.
Fig. 9(a) to Fig. 9(d) are schematic diagrams showing an example of a comparison experiment in which target DNA is detected without fixing a probe DNA on the channel layer.
Fig. 10 is a graph showing a relationship between the top gate voltage V_{TG} and the drain current I_{DS} when the concentration of the target DNA is changed using the comparison experiment in Figs. 9.
Fig. 11(a) is a cross-sectional view (a cross-sectional view along line II-II of Fig. 11(b)) of another example of a configuration of a transistor sensor according to the first embodiment of the present invention and Fig. 11 (b) is a plan view thereof.
Fig. 12 is a cross-sectional view showing yet another example of the configuration of a transistor sensor according to the first embodiment of the present invention.
Fig. 13 is a cross-sectional view showing yet another example of the configuration of a transistor sensor according to the first embodiment of the present invention.
Fig. 14 is a cross-sectional view showing yet another example of the configuration of a transistor sensor according to the first embodiment of the present invention.
Fig. 15 is a cross-sectional view showing yet another example of the configuration of a transistor sensor according to the first embodiment of the present invention.
Fig. 16 is a cross-sectional view showing yet another example of the configuration of a transistor sensor according to the first embodiment of the present invention.
Fig. 17 is a cross-sectional view showing yet another example of the configuration of a transistor sensor according to the first embodiment of the present invention.
Fig. 18(a) to Fig. 18(f) are schematic diagrams showing a step of loading probe DNA for capturing biomaterial which is a measurement target on the transistor sensor in the Examples.
Fig. 19(a) is a graph showing stability evaluation of the transistor sensor in a phosphate buffer in the Examples and Fig. 19(b) is a graph showing stability evaluation in a serum.
Fig. 20 is a graph showing V_{TG}-I_{DS} characteristics measured in a transistor sensor produced in the Examples.
Fig. 21 is a graph in which the target DNA concentration of the sample in the graph of Fig. 20 is plotted as the horizontal axis and the value of V_{TG} when I_{DS} read from the graph of Fig. 20 reaches 1 µA is plotted as the vertical axis.
Fig. 22 is a plan view of the transistor sensor produced in Invention Example 12.
Fig. 23 is a graph of a Cole-Cole plot of the impedance of the solid electrolyte measured in Invention Example 15.

### DESCRIPTION OF EMBODIMENTS

A detailed description will be given below of embodiments of the present invention with reference to the drawings. In the drawings used in the following description, characteristic parts may be enlarged for convenience in order to make the characteristics of the present embodiment easier to understand and the dimensional ratios and the like of each constituent component may differ in practice.

### [Transistor Sensor Configuration]

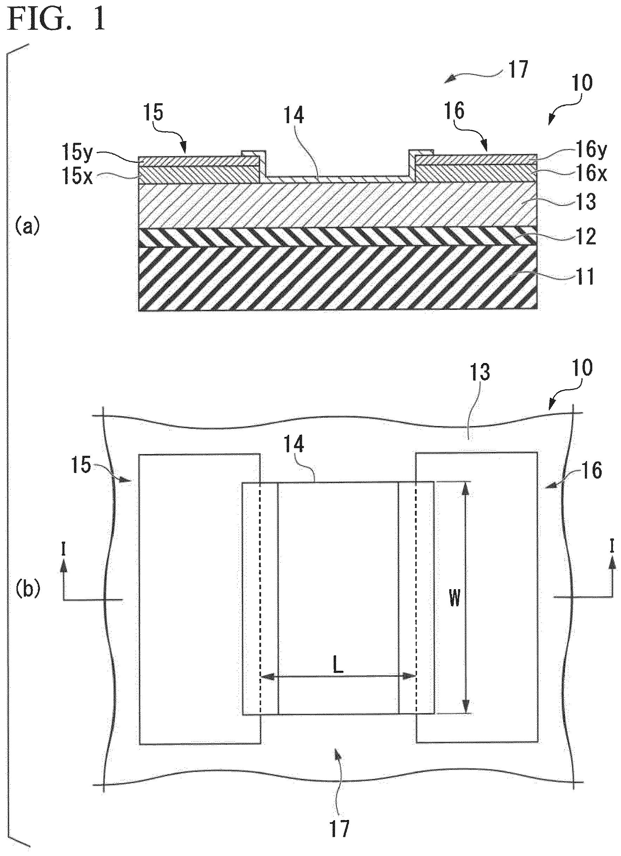

Fig. 1(a) is a cross-sectional view (cross-sectional view along line I-I of Fig. 1(b)) showing an example of a configuration of a transistor sensor according to the first embodiment of the present invention and Fig. 1(b) is a plan view thereof.

As shown in Fig. 1(a) and Fig. 1(b), a transistor sensor 10 is provided with a substrate 11, a back gate electrode (conductive material layer) 12, a gate insulating layer (solid electrolyte layer) 13, a channel layer 14, a source electrode 15, and a drain electrode 16, in that order. In the transistor sensor 10 of the present embodiment, the gate insulating layer (solid electrolyte layer) 13 of a solid electrolyte is formed between the substrate 11 or the back gate electrode (conductive material layer) 12 and the channel layer 14, in particular, between the back gate electrode (conductive material layer) 12 and the channel layer 14. The gate insulating layer (solid electrolyte layer) 13 and the channel layer 14 form an exposed portion 17 which is exposed to the outside. The back gate electrode (conductive material layer) 12 may be removed.

It is possible to form the substrate 11 of various insulating substrates, for example, a highly heat-resistant glass, a SiO₂/Si substrate (that is, a substrate with a silicon oxide film formed on a silicon substrate, also referred to below simply as "substrate"), an alumina (Al₂O₃) substrate, an STO (SrTiO) substrate, an insulating substrate or the like on which an STO (SrTiO) layer is formed on the surface of a Si substrate via a SiO₂ layer and a Ti layer, a semiconductor substrate (for example, an Si substrate, an SiC substrate, a Ge substrate, or the like), and the like. The thickness of the substrate 11 is not particularly limited and is, for example, 10 µm or more and 1 mm or less.

The back gate electrode 12 is a conductive material layer including a conductive material. The back gate electrode (conductive material layer) 12 may be formed solely from a conductive material. For example, it is possible to form the back gate electrode (conductive material layer) 12 of any of metals such as platinum (Pt), gold (Au), silver (Ag), copper (Cu), aluminum (Al), molybdenum (Mo), palladium (Pd), ruthenium (Ru), iridium (Ir), tungsten (W), and titanium (Ti), metal materials such as alloys including these metals, and metal oxides such as indium tin oxide (ITO) and ruthenium oxide (RuO₂). It is possible to form the back gate electrode (conductive material layer) 12 of, for example, a single layer formed of any of the above-described metals, metal materials such as alloys thereof, and metal oxides, or multiple layers in which the above are laminated. The thickness of the back gate electrode (conductive material layer) 12 is not particularly limited and is, for example, 50 nm or more and 200 nm or less. The back gate electrode (conductive material layer) 12 may be electrically insulated except at the gate insulating layer (solid electrolyte layer) 13.

The gate insulating layer 13 is a solid electrolyte layer including a solid electrolyte. The gate insulating layer (solid electrolyte layer) 13 may be formed solely from a solid electrolyte. For example, it is possible to form the gate insulating layer (solid electrolyte layer) 13 of any of a metal oxide including rare earth elements and zirconium (Zr), and a metal oxide including rare earth elements and tantalum (Ta). In addition, the carbon (C) content ratio in the gate insulating layer (solid electrolyte layer) 13 may be 0.5 atom% or more and 15 atom% or less and the hydrogen (H) content ratio in the gate insulating layer (solid electrolyte layer) 13 may be 2 atom% or more and 20 atom% or less. When the gate insulating layer (solid electrolyte layer) 13 is formed of the metal oxide described above and the carbon (C) and hydrogen (H) content ratios in the gate insulating layer (solid electrolyte layer) 13 are both within the above ranges, the combination with the metal oxide described below which forms the channel layer 14 makes the transistor sensor 10 highly sensitive so as to significantly lower the detection limit and increases the detection stability in the presence of moisture or the like.

The gate insulating layer (solid electrolyte layer) 13 preferably has an ionic conductivity of 1 x 10⁻⁸ S/cm or more. The ionic conductivity of the gate insulating layer (solid electrolyte layer) 13 may be 1×10⁻² S/cm or less. Specifically, it is possible to form the gate insulating layer (solid electrolyte layer) 13, for example, of any of the following (A1) to (A5).
(A1) Metal oxide including lanthanum (La) and zirconium (Zr)
(A2) Metal oxide including lanthanum (La) and tantalum (Ta)
(A3) Metal oxide including any metal element selected from the group consisting of cerium (Ce), praseodymium (Pr), neodymium (Nd), samarium (Sm), europium (Eu), gadolinium (Gd), terbium (Tb), dysprosium (Dy), holmium (Ho), erbium (Er), thulium (Tm), ytterbium (Yb), lutetium (Lu), and yttrium (Y), and zirconium (Zr) or tantalum (Ta)
(A4) Metal oxides including at least one metal element selected from the group consisting of hafnium (Hf), zirconium (Zr), and aluminum (Al)
(A5) An oxide containing lanthanum (La), hafnium (Hf), and zirconium (Zr)

For example, in a case (A1) where the gate insulating layer (solid electrolyte layer) 13 is formed of a metal oxide including lanthanum (La) and zirconium (Zr), the atomic number ratio of lanthanum (La) to zirconium (Zr) in the gate insulating layer (solid electrolyte layer) 13 is not particularly limited; however, for example, when lanthanum (La) is 1, zirconium (Zr) is preferably 0.43 or more and 2.33 or less and more preferably 1 or more and 2.33 or less. The atomic number ratio of lanthanum (La) to zirconium (Zr) in the gate insulating layer (solid electrolyte layer) 13 being within the range described above makes it possible to obtain a high sensing performance (typically, high field-effect mobility and high gate capacitor capacitance). In addition, in a case (A2) where the gate insulating layer (solid electrolyte layer) 13 is formed of a metal oxide including lanthanum (La) and tantalum (Ta), the atomic number ratio of lanthanum (La) to tantalum (Ta) in the gate insulating layer (solid electrolyte layer) 13 is also not particularly limited. Furthermore, in a case where the gate insulating layer (solid electrolyte layer) 13 is formed of any of the metal oxides of (A3) to (A5) described above, the atomic number ratio of each metal element is also not particularly limited.

The atomic composition ratios of each of the various elements described above are determined by performing elemental analysis using Rutherford backscattering spectroscopy (RBS method) or the like. In particular, the carbon (C) and hydrogen (H) content ratios are determined by performing elemental analysis using a Pelletron 3 SDH manufactured by National Electrostatics Corporation using Rutherford Backscattering Spectrometry (RBS analysis method), Hydrogen Forward Scattering Spectrometry (HFS analysis method), and nuclear reaction analysis (NRA analysis method).

It is possible to form the gate insulating layer (solid electrolyte layer) 13, for example, of a single layer formed of any of the metal oxides described above. In a case where the gate insulating layer (solid electrolyte layer) 13 is formed of a layer of metal oxide formed of lanthanum (La) and zirconium (Zr), the layer is also referred to as an LZO layer. In addition, in a case where the gate insulating layer (solid electrolyte layer) 13 is formed of a layer of metal oxide formed of lanthanum (La) and tantalum (Ta), the layer is also referred to as an LTO layer.

The thickness of the gate insulating layer (solid electrolyte layer) 13 is not particularly limited and is, for example, 50 nm or more and 300 nm or less. When the thickness of the gate insulating layer (solid electrolyte layer) 13 exceeds 300 nm, there is a possibility that the interface characteristics of the channel layer 14 will be influenced, which is not preferable. On the other hand, when the thickness thereof is less than 50 nm, the thickness is not preferable from the viewpoint of increased leakage current, degradation of the covering property of the film on the substrate, and the like.

The channel layer 14 is an inorganic semiconductor layer including an inorganic semiconductor. The channel layer 14 may be formed solely from an inorganic semiconductor. In addition to Si, which is widely used, for example, it is possible to use zinc oxide and In-containing metal oxides including at least indium (In) as inorganic semiconductors. By forming the channel layer 14 with the zinc oxide or In-containing metal oxide described above, the combination with the metal oxide described above in the gate insulating layer (solid electrolyte layer) 13, in particular, the carbon (C) and hydrogen (H) content ratios in the gate insulating layer (solid electrolyte layer) 13 makes it possible to significantly improve the sensitivity of the transistor sensor 10 and also increase the detection stability in the presence of moisture or the like.

Examples of In-containing metal oxides include (B1) to (B6) oxides below.
(B1) Metal oxides formed of indium (In)
(B2) Metal oxides including indium (In) and tin (Sn)
(B3) Metal oxides including indium (In) and zinc (Zn)
(B4) Metal oxides including indium (In), zirconium (Zr), and zinc (Zn)
(B5) Metal oxides including indium (In) and gallium (Ga)
(B6) Metal oxides including indium (In), zinc (Zn), and gallium (Ga)

For example, in a case (B2) where the channel layer 14 is formed of a metal oxide including indium (In) and tin (Sn), it is possible to include tin (Sn) with an atomic number ratio of 0.001 or more and 0.03 or less when Indium (In) is 1. In a case (B3) where the channel layer 14 is formed of a metal oxide including indium (In) and zinc (Zn), it is possible to include zinc (Zn) with an atomic number ratio of 0.001 or more and 0.75 or less when Indium (In) is 1. In a case (B4) where the channel layer 14 is formed of a metal oxide including indium (In), zirconium (Zr), and zinc (Zn), it is possible to include zinc (Zn) with an atomic number ratio of 0.001 or more and 0.75 or less and zirconium (Zr) with an atomic number ratio of 0.015 or more and 0.075 or less when Indium (In) is 1.

In addition, in a case (B5) where the channel layer 14 is formed of a metal oxide including indium (In) and gallium (Ga), it is possible to include gallium (Ga) with an atomic number ratio of 0.001 or more and 0.75 or less when Indium (In) is 1. In a case (B6) where the channel layer 14 is formed of a metal oxide including indium (In), zinc (Zn), and gallium (Ga), it is possible to include zinc (Zn) with an atomic number ratio of 0.001 or more and 0.75 or less and gallium (Ga) with an atomic number ratio of 0.001 or more and 0.75 or less when Indium (In) is 1.

The metal oxides described above, which are the materials of the channel layer 14, are also confirmed to be in an amorphous phase or a nanocrystalline phase. Accordingly, it is considered that it is possible to obtain a good interface state with the gate insulating layer (solid electrolyte layer) 13, which is in an amorphous phase in contact with the channel layer 14. As a result, it is possible to form the transistor sensor 10 provided with good electrical characteristics.

It is possible to form the channel layer 14, for example, of a single layer formed of the metal oxides described above. The thickness of the channel layer 14 is not particularly limited and is, for example, preferably 5 nm or more and 80 nm or less, from the viewpoint of covering the gate insulating layer (solid electrolyte layer) 13 or the like with a high degree of accuracy and from the viewpoint of facilitating adjustment of the conductivity of the channel layer 14.

In a case (B1) where the channel layer 14 is formed of a layer of metal oxide formed of indium (In), the layer is also referred to as an InO layer. In addition, in a case (B2) where the channel layer 14 is formed of a layer of metal oxide including indium (In) and tin (Sn), the layer is also referred to an indium tin oxide (ITO) layer. In a case (B3) where the channel layer 14 is formed of a layer of metal oxide including indium (In) and zinc (Zn), the layer is also referred to an IZO layer. In a case (B4) where the channel layer 14 is formed of a layer of metal oxide including indium (In), zinc (Zn), and zirconium (Zr), the layer is also referred to as a ZIZO layer.

A width W (Fig. 1(b)) of the channel layer 14 being larger is advantageous from the viewpoint of noise and is, for example, 100 µm or more and 1000 µm or less as an example. A length L of the channel layer 14 is, for example, 50 µm or more and 200 µm or less.

In addition, in the present embodiment, the channel layer 14 has a thinner film thickness than both the source electrode 15 and the drain electrode 16, but is not limited thereto and may have the same film thickness as the source electrode 15 and the drain electrode 16 or may have a thicker film thickness.

The source electrode 15 and the drain electrode 16 are arranged, for example, on both sides of the channel layer 14 on the gate insulating layer (solid electrolyte layer) 13. The source electrode 15 and the drain electrode 16 are not particularly limited and are able to be formed of, for example, any of a high-melting-point metal such as platinum (Pt), a metal material such as an alloy including the high-melting-point metal, and a metal oxide such as indium tin oxide (ITO) and ruthenium oxide (RuO₂). It is possible to form the source electrode 15 of a single layer formed of any of the above-described metals, metal materials such as alloys thereof, and metal oxides, or of multiple layers formed of laminated layers thereof. It is also possible to form the drain electrode 16 of a single layer formed of any of the above-described metals, metal materials such as alloys thereof, and metal oxides, or of multiple layers formed of laminated layers thereof. In the present embodiment, the source electrode 15 is formed of multiple layers in which a first source electrode 15x and a second source electrode 15y are laminated. In addition, the drain electrode 16 is formed of multiple layers in which a first drain electrode 16x and a second drain electrode 16y are laminated. The thickness of the source electrode 15 and the thickness of the drain electrode 16 are not particularly limited and are, for example, 50 nm or more and 1000 nm or less.

### [Method for Manufacturing Transistor Sensor]

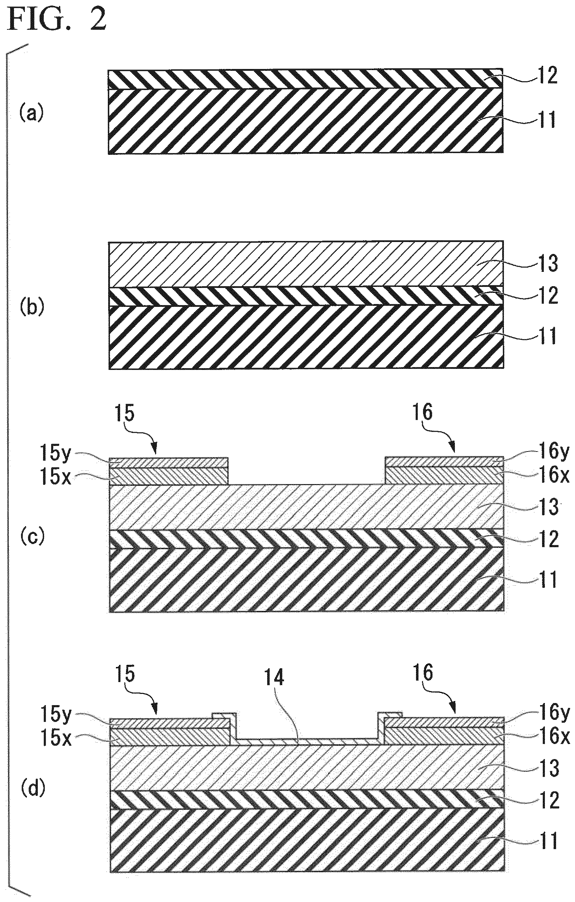

Fig. 2(a) to Fig. 2(d) are cross-sectional views showing an example of steps of a method for manufacturing the transistor sensor 10 of Fig. 1(a).

### (1) Formation of Back Gate Electrode (Conductive Material Layer)

First, as shown in Fig. 2(a), the back gate electrode (conductive material layer) 12 is formed on the SiO₂/Si substrate (also referred to below simply as "substrate") 11, which is the base material, by a known sputtering method, photolithography method, and etching method.

### (2) Formation of Gate Insulating Layer (Solid Electrolyte Layer)

Next, on the back gate electrode (conductive material layer) 12, by a known spin coating method, a precursor layer for the gate insulating layer (solid electrolyte layer) is formed with starting materials of a precursor solution for the gate insulating layer (solid electrolyte layer) having a precursor including rare earth elements and a precursor including zirconium (Zr) as a solute, or a precursor solution for the gate insulating layer (solid electrolyte layer) having a precursor including rare earth elements and a precursor including tantalum (Ta) as a solute. In the present embodiment, for example, a precursor layer for the gate insulating layer (solid electrolyte layer) is formed with starting materials of a precursor solution for the gate insulating layer (solid electrolyte layer) having a precursor including lanthanum (La) and a precursor including zirconium (Zr) as solute.

At this time, the precursor solution for the gate insulating layer (solid electrolyte layer) may be adjusted such that the carbon (C) content ratio in the gate insulating layer (solid electrolyte layer) 13 to be finally formed is 0.5 atom% or more and 15 atom% or less and the hydrogen (H) content ratio in the gate insulating layer (solid electrolyte layer) 13 is 2 atom% or more and 20 atom% or less. Specifically, the precursor solution for the gate insulating layer (solid electrolyte layer) may be adjusted according to the methods shown in (2-1) to (2-3) below.
(2-1) Lanthanum acetate is dissolved in propionic acid by heating at 110°C for 30 minutes to obtain a solution of 0.2 mol/kg.
(2-2) Zirconium butoxide is dissolved in propionic acid by heating at 110°C for 30 minutes to obtain a solution of 0.2 mol/kg.
(2-3) Each of the solutions of (2-1) and (2-2) described above are mixed at room temperature.

From the viewpoint of further improving the characteristics of the transistor sensor, the carbon (C) content ratio described above may be 1 atom% or more and 10 atom% or less and the hydrogen (H) content ratio in the gate insulating layer (solid electrolyte layer) 13 may be 5 atom% or more and 18 atom% or less.

Examples of a precursor including lanthanum (La) include lanthanum acetate. As other examples, lanthanum nitrate, lanthanum chloride, or various lanthanum alkoxides (for example, lanthanum isopropoxide, lanthanum butoxide, lanthanum ethoxide, and lanthanum methoxyethoxide) may be adopted.

In addition, an example of a precursor including zirconium (Zr) is zirconium butoxide. As other examples, zirconium nitrate, zirconium chloride, or various other zirconium alkoxides (for example, zirconium isopropoxide, zirconium butoxide, zirconium ethoxide, and zirconium methoxyethoxide) may be adopted.

In addition, an example of a precursor including tantalum (Ta) is tantalum butoxide. As other examples, tantalum nitrate, tantalum chloride, or various other tantalum alkoxides (for example, tantalum isopropoxide, tantalum butoxide, tantalum ethoxide, and tantalum methoxyethoxide) may be adopted.

Examples of precursors including cerium (Ce) include Ce octylate, Ce nitrate, and the like. Examples of precursors including praseodymium (Pr) include Pr octylate, Pr nitrate, and the like. Examples of precursors including neodymium (Nd) include Nd octylate, Nd nitrate, and the like. Examples of precursors including samarium (Sm) include Sm octylate, Sm nitrate, and the like. Examples of precursors including europium (Eu) include Eu octylate, Eu nitrate, and the like. Examples of precursors including gadolinium (Gd) include Gd octylate, Gd nitrate, and the like. Examples of precursors including terbium (Tb) include Tb octylate, Tb nitrate, and the like. Examples of precursors including dysprosium (Dy) include Dy octylate, Dy nitrate, and the like. Examples of precursors including holmium (Ho) include Ho octylate, Ho nitrate, and the like. Examples of precursors including erbium (Er) include Er octylate, Er nitrate, and the like. Examples of precursors including thulium (Tm) include Tm octylate, Tm nitrate, and the like. Examples of precursors including ytterbium (Yb) include Yb octylate, Yb nitrate, and the like. Examples of precursors including lutetium (Lu) include Lu octylate, Lu nitrate, and the like. Examples of precursors including yttrium (Y) include Y octylate, Y nitrate, and the like.

In addition, examples of precursors including hafnium (Hf) include Hf octylate, Hf nitrate, Hf butoxide, and the like. Examples of precursors including aluminum (Al) include Al octylate, Al nitrate, Al butoxide, and the like.

Thereafter, as a pre-firing, heating is carried out at 80°C or higher and 250°C or lower for a predetermined time. This pre-firing sufficiently evaporates the solvent in the precursor layer for the gate insulating layer (solid electrolyte layer) and also makes it possible to form a gel state (considered to be a state before thermal decomposition and in which organic chains remain) which is preferable for expressing characteristics that enable future plastic deformation. From the viewpoint of realizing the viewpoint described above with a higher degree of certainty, the pre-firing temperature is preferably 100°C or higher and 250°C or lower. In addition, this temperature range is also the preferable temperature range for the pre-firing of other materials.

This pre-firing is performed in an oxygen atmosphere or in air (also referred to below collectively as "oxygen-containing atmosphere"). In the present embodiment, the formation and pre-firing of the precursor layer for the gate insulating layer (solid electrolyte layer) by the spin coating method described above is repeated a plurality of times in order to finally obtain a sufficient thickness (for example, 120 nm) of the gate insulating layer (solid electrolyte layer) 13.

Further thereafter, as a main firing, the precursor layer for the gate insulating layer (solid electrolyte layer) is heated in an oxygen-containing atmosphere (for example, an oxygen content of 100% by volume without being limited thereto; the same also applies below to "oxygen-containing atmosphere") in a range of 250°C or higher and 450°C or lower for a predetermined time. Due to this, as shown in Fig. 2(b), the gate insulating layer (solid electrolyte layer) 13, which is any of a metal oxide including rare earth elements and zirconium (Zr) and a metal oxide including rare earth elements and tantalum (Ta), is formed on the back gate electrode (conductive material layer) 12.

### (3) Formation of Source Electrode and Drain Electrode

Thereafter, a resist film (not shown) patterned by a known photolithography method is formed on the gate insulating layer (solid electrolyte layer) 13. Then, the first electrode layer and second electrode layer are formed on the gate insulating layer (solid electrolyte layer) 13 and the resist film by a known sputtering method. In the present embodiment, for example, an ITO layer and a Pt layer are formed on the gate insulating layer (solid electrolyte layer) 13 in that order to form the first electrode layer formed of an ITO layer and a Pt layer and the second electrode layer formed of an ITO layer and a Pt layer. In a case of forming the ITO layer, for example, it is possible to use an ITO layer target material containing 5 wt% tin oxide (SnO₂). Thereafter, the resist film is removed and, as shown in Fig. 2(c), the source electrode 15 formed of the first source electrode (ITO layer) 15x and the second source electrode (Pt) 15y, and the drain electrode 16 formed of the first drain electrode (ITO layer) 16x and the second drain electrode (Pt) 16y are formed on the gate insulating layer (solid electrolyte layer) 13.

### (4) Formation of Channel Layer

Further thereafter, a precursor layer for the channel layer is formed on the gate insulating layer (solid electrolyte layer) 13 by a known spin coating method, with a starting material of a precursor solution for the channel layer having at least indium (In) as a solute. In the present embodiment, for example, a precursor layer for the channel layer is formed with a starting material of the precursor solution for the channel layer shown in any of (C1) to (C6) below.
(C1) A precursor solution for the channel layer with a precursor including indium (In) as a solute
(C2) A precursor solution for the channel layer with a precursor including indium (In) and a precursor including tin (Sn) as a solute
(C3) A precursor solution for the channel layer with a precursor including indium (In) and a precursor including zinc (Zn) as a solute
(C4) A precursor solution for the channel layer with a precursor including indium (In), a precursor including zirconium (Zr), and a precursor including zinc (Zn) as a solute
(C5) A precursor solution for the channel layer with a precursor including indium (In) and a precursor including gallium (Ga) as a solute
(C6) A precursor solution for the channel layer with a precursor including indium (In), a precursor including zinc (Zn), and a precursor including gallium (Ga) as a solute

In addition, the precursor solution for the channel layer described above further includes at least one auxiliary firing agent selected from the group of acetylacetonate, urea, and ammonium acetate, and an oxidizing agent. Example of oxidizing agents are nitrate, peroxide, or perchlorate.

Examples of a precursor including indium (In) include indium nitrate. As other examples, indium acetylacetonate, indium acetate, indium chloride, or various indium alkoxides (for example, indium isopropoxide, indium butoxide, indium ethoxide, and indium methoxyethoxide) may be adopted.

An example of a precursor including tin (Sn) is tin chloride. As other examples, tin nitrate, tin acetate, or various tin alkoxides (for example, tin isopropoxide, tin butoxide, tin ethoxide, and tin methoxyethoxide) may be adopted.

In addition, examples of a precursor including zinc include (Zn)zinc chloride. As other examples, zinc nitrate, zinc acetate, or various zinc alkoxides (for example, zinc isopropoxide, zinc butoxide, zinc ethoxide, and zinc methoxyethoxide) may be adopted.

Example of a precursor including zirconium (Zr) include zirconium butoxide. As other examples, zirconium nitrate, zirconium chloride, or various other zirconium alkoxides (for example, zirconium isopropoxide, zirconium butoxide, zirconium ethoxide, and zirconium methoxyethoxide) may be adopted.

Examples of a precursor including gallium (Ga) include gallium isopropoxide. As other examples, gallium methoxide, gallium ethoxide, gallium n-propoxide, gallium butoxide, gallium nitrate, and gallium octylate may be adopted.

Thereafter, as a pre-firing, the precursor layer for the channel layer is heated in the range of 80°C or higher and 250°C or lower for a predetermined time. Thereafter, as the main firing, the precursor layer for the channel layer is heated in an oxygen-containing atmosphere for a predetermined time in a range of 250°C or higher and 400°C or lower. Due to this, as shown in Fig. 2(d), the channel layer 14, which is a metal oxide including at least indium (In), is formed on the gate insulating layer (solid electrolyte layer) 13.

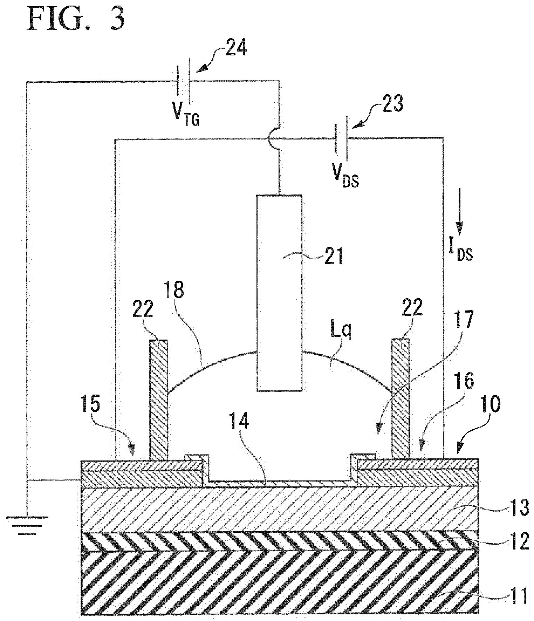

Fig. 3 is a schematic diagram showing a method for detecting DNA using the transistor sensor 10 of Fig. 1.

As shown in Fig. 3, first, as necessary, wall portions 22 are placed so as to surround the exposed portion 17 (channel layer 14) of the transistor sensor 10. Due to this, it is possible to form a liquid holding portion 18 able to hold a liquid Lq including a biomaterial, at least in an upper portion of the channel layer 14 and inside the wall portions 22. The wall portions 22 are arranged, for example, so as to surround the channel layer 14, at least a portion of the source electrode 15, and at least a portion of the drain electrode 16.

Next, a probe molecule for capturing a specific biomaterial (biomolecule) is fixed on the channel layer 14. The probe molecule is not particularly limited and is, for example, an antibody or single-stranded DNA. At this time, the probe molecules may be fixed not only on the channel layer 14 but also on the gate insulating layer (solid electrolyte layer) 13.

In this manner, on the channel layer 14, the transistor sensor 10 has a capturing field which indirectly captures the biomaterial which is the measurement target (for example, target DNA). It is also possible to refer to this capturing field as a capturing surface which captures the biomaterial and the capturing surface is formed on the surface of the channel layer 14. A probe molecule (for example, probe DNA) for capturing the biomaterial described above is fixed to the capturing field or the capturing surface.

The method for fixing the probe molecule described above is an example and the probe molecule may be fixed on the transistor sensor 10 by methods other than the above. In addition, a capturing field may be formed on the channel layer 14 by methods other than the above.

After the biomaterial is fixed to the probe molecule as described above, it is preferable to apply a blocking treatment for suppressing non-specific binding on the channel layer 14 (or on the channel layer 14 and the gate insulating layer (solid electrolyte layer) 13).

Next, a liquid including a biomaterial is arranged in contact with the channel layer 14 (or the channel layer 14 and the gate insulating layer (solid electrolyte layer) 13) and the biomaterial which is the measurement target among the biomaterials included in the liquid is specifically bound to the probe molecules described above. The biomaterial is not particularly limited and is, for example, a nucleic acid such as DNA or mRNA. In the present embodiment, for example, the liquid Lq (a mixed solution of a buffer solution and a serum including nucleic acid, or the like) including a biomaterial is supplied on the channel layer 14 and the liquid Lq is held in the liquid holding portion 18.

Thereafter, it is preferable to wash the biomaterial that was not bound to the probe molecule, the biomaterial that is not a measurement target, the biomaterial that is non-specifically bound, or the like, with a washing solution. After washing, a measurement solution such as a buffer solution is supplied on the channel layer 14 (or on the channel layer 14 and the gate insulating layer (solid electrolyte layer) 13). Due to this, it is possible to electrically connect the channel layer 14 and the reference electrode 21 via the measurement solution.

Next, the reference electrode 21 is inserted into the liquid Lq to electrically connect the reference electrode 21 and the source electrode 15 and the voltage V_{TG} is applied between the reference electrode 21 and the source electrode 15 using a second voltage supply portion 24. Thereafter, the source electrode 15 and the drain electrode 16 of the transistor sensor 10 are electrically connected, a voltage V_{DS} is applied between the source electrode 15 and the drain electrode 16 using a first voltage supply portion 23, and the current I_{DS} flowing between the source electrode 15 and the drain electrode 16 is measured. Then, the biomaterial is detected based on the voltage V_{TG} (also referred to below as the top gate voltage) between the reference electrode 21 and the source electrode 15 and the current I_{DS} (also referred to below as the drain current) between the source electrode 15 and the drain electrode 16.

In this transistor sensor 10, a so-called top-gate structure is formed and the voltage V_{TG} is applied to the channel layer 14 from the reference electrode 21. When a biomaterial which is a measurement target specifically binds to a probe molecule captured on the channel layer 14, the current I_{DS} changes due to the change in electric charge and the change in the V_{TG}-I_{DS} characteristic is detected according to the change in the current I_{DS}. Due to this, it is possible to detect the amount of the specific biomaterial which is a measurement target.

In the present embodiment, a voltage is not applied to the back gate electrode (conductive material layer) 12; however, without being limited thereto, a voltage V_{BG} may be applied to the back gate electrode (conductive material layer) 12 using a third voltage supply portion (not shown). In addition, the back gate electrode (conductive material layer) 12 may be formed of a floating electrode. Further, the back gate electrode (conductive material layer) 12 itself may not be provided.

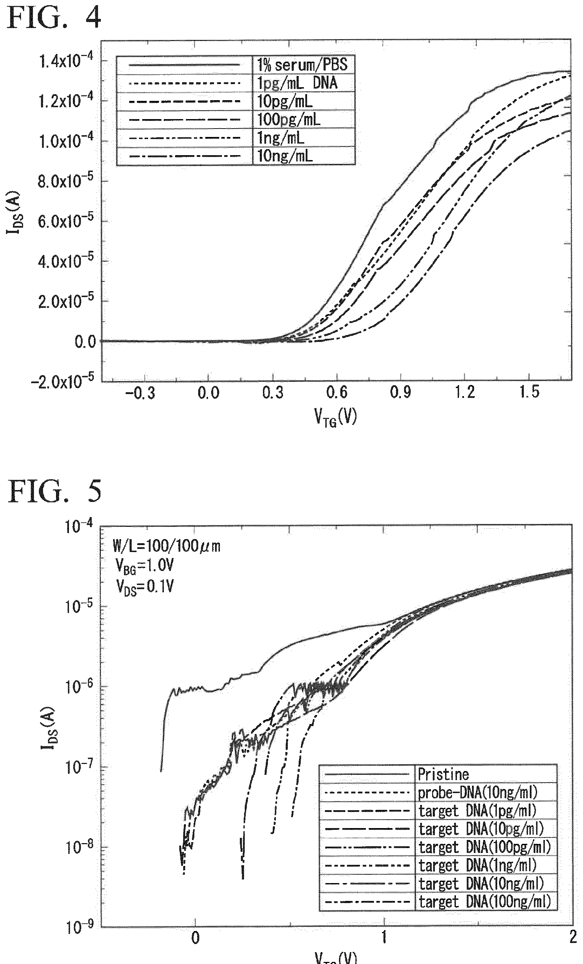

Fig. 4 is a graph showing the V_{TG}-I_{DS} characteristics of the transistor sensor 10 of Fig. 1. In Fig. 4, as an example of the transistor sensor 10, the thin-film transistor of Invention Example 1 is produced under the conditions shown in Table 1 and the V_{TG}-I_{DS} characteristics obtained from the measured values of voltage V_{TG} and current I_{DS} by the method described above are shown. A description will be given of the method for manufacturing the transistor sensor of Invention Example 1 in the Examples below.

**[Table 1]**

| | | Invention Example 1 | TFT sensor of related art |
|---|---|---|---|
| Substrate | Material | SiO₂/Si | SiO₂/Si |
| | Thickness | 500mn(SiO₂) | 500nm(SiO₂) |
| Back gate electrode (conductive material layer) | Material | Pt/Ti | Pt/Ti |
| | Thickness (Pt/Ti) | 100nm/ 10nm | 100nm/10nm |
| Gate insulating layer (solid electrolyte layer) | Material | LZO | LZO |
| | Role of gate insulating layer | Solid electrolyte layer | Insulating layer |
| | Element ratio (La:Zr) | 5:5(1:1) | 5:5(1:1) |
| | Thickness | 120nm | 120nm |
| | Heating temperature | 400°C | 550°C |
| | Carbon (C) content ratio | 2.3atom% | 0.4atom% |
| | Hydrogen (H) content ratio | 10.8atom% | 1.8atom% |
| Channel layer | Material | In₂O₃ | In₂O₃ |
| | Thickness | 20nm | 20nm |
| | Heating temperature | 300°C | 500°C |
| Source electrode | Material | Pt/ITO | Pt/ITO |
| | Thickness (Pt/ITO) | 100nm/50nm | 100nm/50nm |
| Drain electrode | Material | Pt/ITO | Pt/ITO |
| | Thickness (Pt/ITO) | 100nm/50nm | 100nm/50nm |

As shown in Fig. 4, in the transistor sensor 10 of the present embodiment, when the heating temperature during the formation of the gate insulating layer (solid electrolyte layer) 13 and the heating temperature during the formation of the channel layer 14 are 300°C and 400°C, each, and the carbon (C) and the hydrogen (H) content ratios in the gate insulating layer (solid electrolyte layer) 13 are 2.3 atom% and 10.8 atom%, respectively, it was understood that, with the top gate voltage V_{TG} being in the range of 0.6 V to 1.5 V, the V_{TG}-I_{DS} curve shifted to the lower voltage side and responded almost linearly with respect to the concentration of target DNA. In particular, even in a case where the target DNA was 1 pg/mL, the drain current I_{DS} increased linearly along with the increase of the top gate voltage V_{TG}. From this, it is understood that, in the transistor sensor 10, the detection stability in liquid is high even in a region with a low concentration of the target DNA and the concentration range in which it is possible to detect the target DNA is in a range of at least 1 pg/mL or more.

Fig. 5 is a graph showing the V_{TG}-I_{DS} characteristics of a transistor sensor of the related art. In Fig. 5, as an example of a transistor sensor of the related art, a transistor (TFT sensor) is produced under the conditions shown in Table 1 and the V_{TG}-I_{DS} characteristics obtained from the measurement values of the voltage V_{TG} and the current I_{DS} by the method described above are shown. A description will be given below of the method for manufacturing the TFT sensor of the related art in the Examples below.

As shown in Fig. 5, in the transistor sensor of the related art, the heating temperature during the formation of the gate insulating layer (insulator layer) and heating temperature during the formation of the channel layer were 550°C and 500°C, respectively, the carbon (C) and hydrogen (H) content ratios in the gate insulating layer (insulator layer) were 0.4 atom% and 1.8 atom%, respectively, and variations occurred in the V_{TG}-I_{DS} curve with respect to the concentration of the target DNA. In particular, when the concentration of the target DNA was 1 ng/mL, 100 pg/mL, 10 pg/mL, and 1 pg/mL, there were disturbances in the V_{TG}-I_{DS} curve. From this, it is understood that, with the transistor sensor of the related art, detection of the target DNA in liquid at 1 ng/mL or less is unstable and the concentration range in which it is possible to detect the target DNA is in a range of greater than 1 ng/mL.

That is, it is understood that, while the detection limit of the target DNA in the transistor sensor of the related art (TFT sensor of the related art) is approximately 1 ng/mL, the detection limit of the target DNA in the transistor sensor 10 of the present embodiment (Invention Example 1) is 1 pg/mL and the detection limit of the transistor sensor 10 is approximately 1000 times or more higher than the detection limit of the transistor sensor of the related art.

In this manner, it is assumed that the transistor sensor 10 of the present embodiment may be able to achieve extremely sensitive detection and measurement by setting each of the carbon (C) and hydrogen (H) content ratios in the gate insulating layer (solid electrolyte layer) 13 within specific ranges.

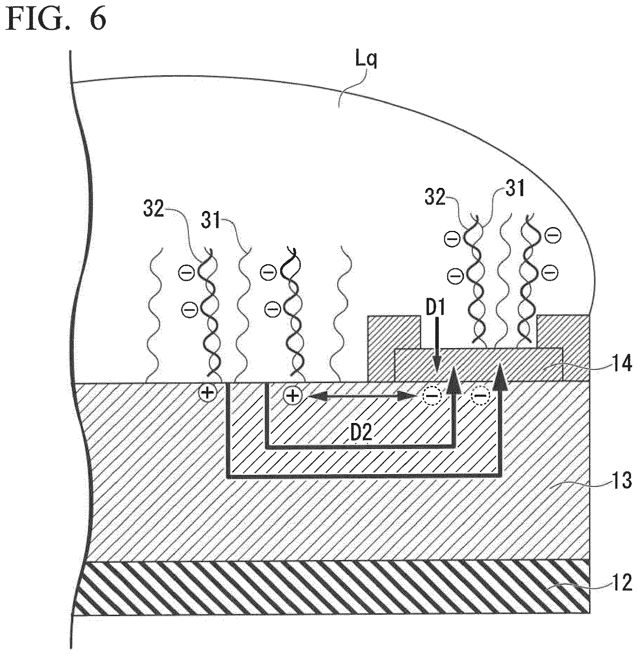

In addition, in the transistor sensor 10 of the present embodiment, having the gate insulating layer (solid electrolyte layer) 13 further improves the sensitivity with respect to target DNA 32 and the detection stability by widening the detectable range of the target DNA. A description will be given of the action of the gate insulating layer (solid electrolyte layer) with reference to Fig. 6.

Fig. 6 is a cross-sectional view of a transistor sensor showing the action of the gate insulating layer (solid electrolyte layer) and the back gate electrode (conductive material layer).

In the transistor sensor of the related art, when probe DNA 31 fixed on the surface of the channel layer 14 captures the target DNA 32, the current flowing through the channel layer 14 or the electrical characteristics such as I-V characteristics change due to the electric field created by the charge of the target DNA 32. In such a case, it is possible for only the target DNA 32 captured on the surface of the channel layer 14 to contribute to the change. On the other hand, in the transistor sensor 10 of the present embodiment, along with an electric field (arrow D1) created by the charge of the target DNA 32 captured by the probe DNA 31 fixed on the surface of the channel layer 14, ions move inside the gate insulating layer (solid electrolyte layer) 13 such that an electric field (arrow D2) created by the charge of the target DNA 32 captured by the probe DNA 31 fixed on the surface of the gate insulating layer (solid electrolyte layer) 13 is transmitted to the channel layer 14 and is able to contribute to the change in electrical characteristics (antenna effect). Due to this, the larger electric field created by the charge of the target DNA 32 accumulates in the channel layer 14, thereby improving sensitivity and stability. In addition, it is also possible for the transistor sensor 10 of the present embodiment to have the back gate electrode (conductive material layer) 12 having electrical conductivity in contact with the gate insulating layer (solid electrolyte layer) 13. This has the effect of transferring the electric field created by the charge of the captured target DNA 32 faster, farther, and in greater amounts to the channel layer.

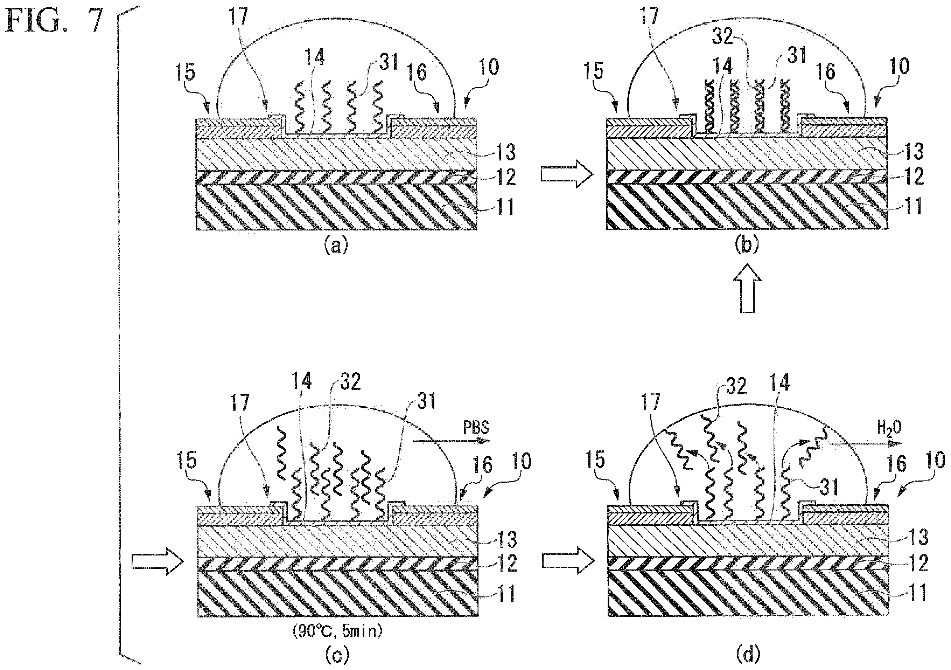

Fig. 7(a) to Fig. 7(d) are schematic diagrams showing an example of a detection method in which the transistor sensor 10 of Figs. 1 (a) and 1(b) is used to repeatedly detect target DNA.

A liquid adjusted so that the concentration of the probe DNA is a predetermined value is supplied on the channel layer 14 and the probe DNA 31 is fixed on the channel layer 14 by the method shown in Fig. 3 (Fig. 7(a)). When fixing the probe DNA 31 on the channel layer 14, the probe DNA 31 may also be fixed on the gate insulating layer (solid electrolyte layer) 13. Next, the target DNA 32 is captured by hybridization (Fig. 7(b)). Thereafter, the V_{TG}-I_{DS} characteristics are measured by the method shown in Fig. 3.

In a case where repeated measurements are performed, thereafter, by supplying a buffer solution such as PBS on the channel layer 14 and heating, for example, at 90°C for 5 minutes, the target DNA 32 may then be released into the buffer solution (Fig. 7(c)). Furthermore, the top of the channel layer 14 may be washed with pure water to remove the target DNA 32 from the channel layer 14 (Fig. 7(d)). In such a case, it is possible to measure the V_{TG}-I_{DS} characteristics in the same manner as described above by supplying liquid adjusted such that the probe DNA 31 concentrations are different onto the channel layer 14 (Fig. 7(a) and Fig. 7(b)). By repeating these steps, it is possible to use a plurality of liquids with different concentrations of the probe DNA 31 and to measure the top gate voltage V_{TG} and drain current I_{DS} with respect to each concentration.

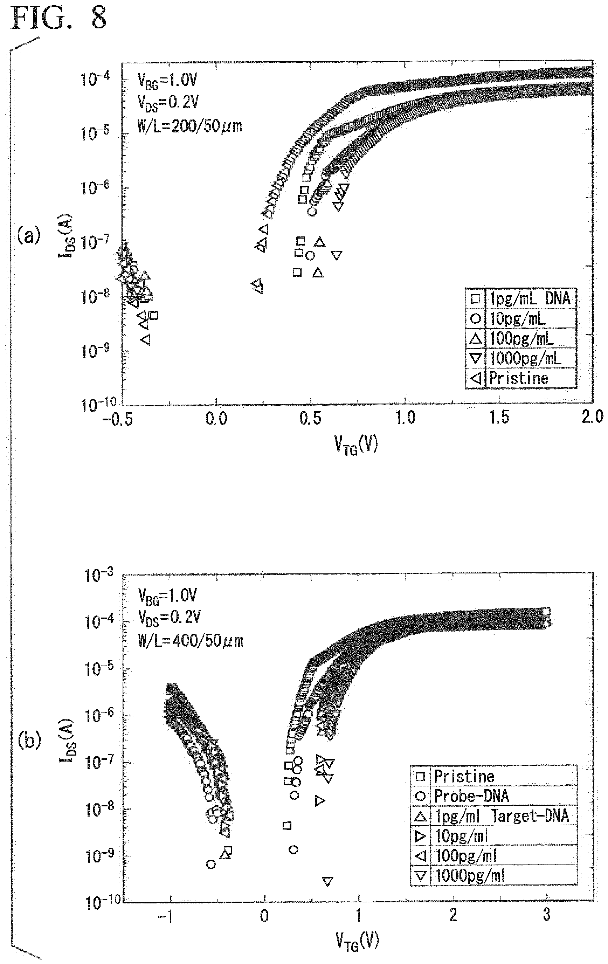

Fig. 8(a) and Fig. 8(b) are graphs showing the relationship between the top gate voltage V_{TG} and the drain current I_{DS} when the width W of the channel layer 14 is changed in the transistor sensor 10 of Fig. 1. In Fig. 8(a), as an example, the transistor sensor 10, in which the gate insulating layer (solid electrolyte layer) 13 is formed of LZO and the channel layer 14 is formed of indium oxide (In₂O₃), is used. In addition, the width W of the channel layer 14 is 200 µm, the length L is 50 µm, and the concentrations of the target DNA are 1 pg/mL, 10 pg/mL, 100 pg/mL, and 1000 pg/mL. In Fig. 8(b), the measurements are carried out under the same conditions as in Fig. 8(a), except that the width W of the channel layer 14 is changed to 400 µm.

As shown in Fig. 8(a), it is understood that in a range where the top gate voltage V_{TG} is 0 or more (0 ≤ V_{TG}), the V_{TG}-I_{DS} characteristic shifts overall to the lower right along with the increase of the concentration of the target DNA. In particular, the V_{TG}-I_{DS} characteristic in a case where the concentration of the target DNA is an extremely small amount of 1 pg/mL shows the same trend as the V_{TG}-I_{DS} characteristic at other concentrations and the drain current I_{DS} increases along with the increase of the top gate voltage V_{TG}.

In addition, as shown in Fig. 8(b), even in a case where the width W of the channel layer 14 is twice the width W of the channel layer 14 in Fig. 8(a), it is understood that, in a range where the top gate voltage V_{TG} is 0 or more (0 ≤ V_{TG}), the V_{TG}-I_{DS} characteristic shifts overall to the lower right along with the increase of the concentration of the target DNA.

Thus, from the results of Fig. 8(a) and Fig. 8(b), it is understood that the hybridization detection range is at least 1 pg/mL to 1 ng/mL in the transistor sensor 10. It is assumed that the extremely high detection limit of 1 pg/mL for the transistor sensor 10 formed as described above is due to the high product of, for example, the electron mobility and gate capacitance.

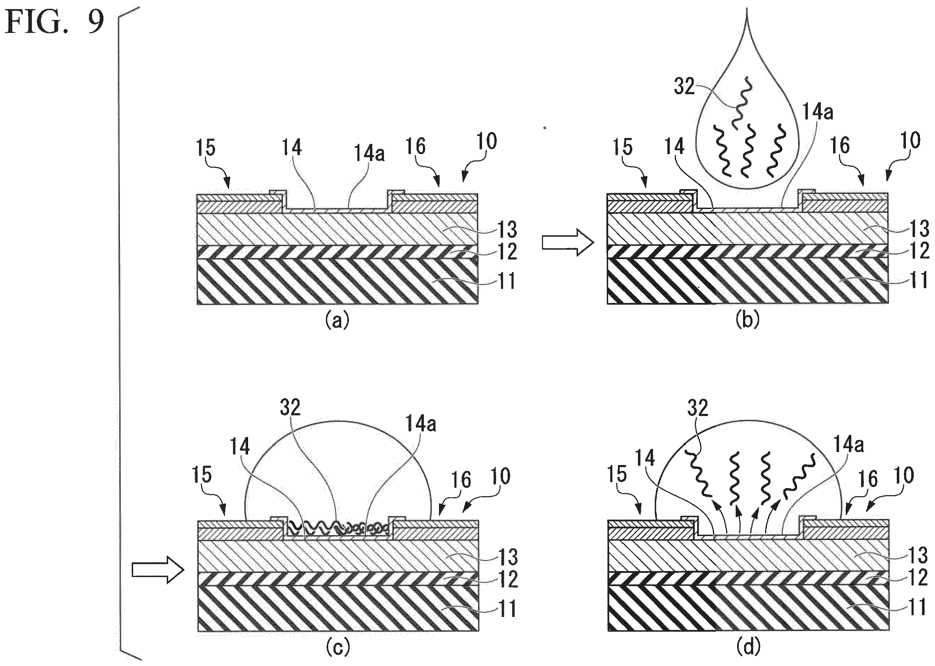

Fig. 9(a) to Fig. 9(d) are schematic diagrams showing an example of a comparison experiment in which target DNA is detected without fixing probe DNA on the channel layer 14.

As a comparison experiment, for example, the transistor sensor 10 is produced by the same method as the method shown in Figs. 2 (Fig. 9(a)) and a liquid including a biomaterial such as the target DNA 32 is supplied onto the channel layer 14 (or onto the channel layer 14 and the gate insulating layer (solid electrolyte layer) 13) using a pipette or the like (Fig. 9(b)). Thereafter, as necessary, the biomaterial is cultured for, for example, 30 minutes. At this time, the biomaterial, such as the target DNA 32, is adsorbed on the surface 14a of the channel layer 14 by physical adsorption (Fig. 9(c)).

The transistor sensor 10 formed as described above does not have a capturing field on the channel layer 14 to directly capture biomaterial. Thus, when the top of the channel layer 14 is subsequently washed with pure water or the like, biomaterials such as the target DNA 32 are easily desorbed from the surface 14a of the channel layer 14 (Fig. 9(d)).

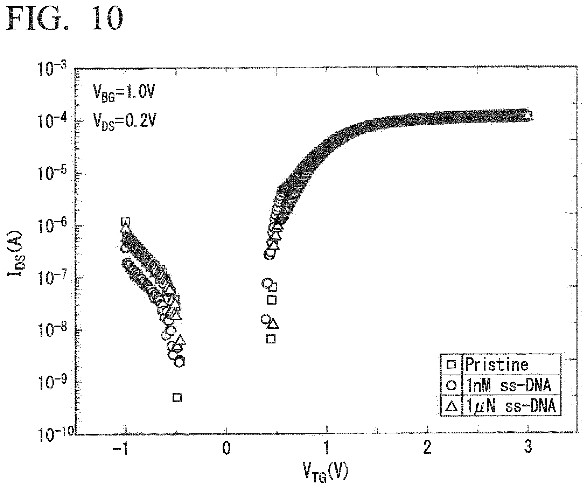

Fig. 10 is a graph showing the relationship between the top gate voltage V_{TG} and the drain current I_{DS} when the concentration of the target DNA is changed using the comparison experiment of Figs. 9. In Fig. 10, as an example, the transistor sensor 10, in which the gate insulating layer (solid electrolyte layer) 13 is formed of LZO and the channel layer 14 is formed of indium oxide (In₂O₃), is used. In addition, the concentrations of the target DNA are 1 nM/mL and 1 µM/mL.

As shown in Fig. 10, the V_{TG}-I_{DS} characteristics in a case where the concentration of the target DNA is 1 nM/mL are almost unchanged from the V_{TG}-I_{DS} characteristics in a case where a liquid including no target DNA is used. In addition, similarly, in a case where the concentration of the target DNA is 1nM/mL, almost no change in the V_{TG}-I_{DS} characteristics is seen. It is possible to confirm that, by washing over the channel layer 14, non-specifically bound biomaterials and the like are removed from the channel layer 14 and, as a result, non-specifically bound biomaterials or the like are not detected and only specifically bound biomaterials are detected.

As described above, according to the present embodiment, since the channel layer 14 includes an inorganic semiconductor and the gate insulating layer (solid electrolyte layer) 13 includes an inorganic solid electrolyte, it is possible to achieve a low detection limit value with high sensitivity without the need for amplification, to realize high stability detection, and, furthermore, to detect biomaterials rapidly and easily at low cost. In a case where the gate insulating layer (solid electrolyte layer) 13 is formed of any one of metal oxides including rare earth elements and zirconium (Zr) and metal oxides including rare earth elements and tantalum (Ta), the channel layer 14 is formed of a metal oxide including at least indium (In), the carbon (C) content ratio in the gate insulating layer (solid electrolyte layer) 13 is 0.5 atom% or more and 15 atom% or less, and the hydrogen (H) content ratio in the gate insulating layer (solid electrolyte layer) 13 is 2 atom% or more and 20 atom% or less, in particular, the detection limit value is low with high sensitivity, thus, the detection of target DNA is possible on a single molecule basis in a shorter time than in the related art and it is possible to realize highly precise detection in a short time when analyzing infectious diseases, novel cancer markers, individual genes, exosomes, mRNA in leukocytes, cell-free RNA/DNA, and the like. In addition, in the medical field, it is possible to detect biomaterials related to a patient during medical examination of the patient and to provide rapid and appropriate diagnosis and carry out treatment based on the detection results. Furthermore, according to the manufacturing method described above, it is possible to provide the transistor sensor 10 with excellent industrial efficiency and mass productivity.

In addition, according to the present embodiment, a liquid including a biomaterial such as target DNA is supplied on the channel layer 14 of the transistor sensor 10, a voltage is applied between the reference electrode 21 inserted in the liquid including the biomaterial and the source electrode 15 of the transistor sensor 10, the current between the source electrode 15 and the drain electrode 16 of the transistor sensor 10 is measured, and the biomaterial is detected based on the voltage between the reference electrode 21 and the source electrode 15 and the current between the source electrode 15 and the drain electrode 16, thus, the present detection method makes it possible to detect biomaterials in a short time and with high sensitivity, with simple operation as in the related art and at a cost equivalent to that of the related art.

In addition, it is also possible to provide a biomaterial detection apparatus having a multi-structure or array structure in which a plurality of the transistor sensors 10 of the present embodiment are arranged. Furthermore, it is possible to provide a biomaterial detection apparatus which is provided with both the transistor sensor 10 of the present embodiment and other electrochemical sensors such as known carbon sensors. Due to this, it is possible to realize highly precise detection in a short time, to detect both biomaterials such as mRNA/DNA and other biomaterials such as biomarkers in the same period or simultaneously, and to carry out analysis from various viewpoints using the detection results of a plurality of biomaterials obtained in a single detection operation.

Although embodiments of the present invention are described in detail above, the present invention is not limited to the embodiments described above and various variations and changes are possible within the scope of the gist of the present invention as described in the claims.

For example, in the transistor sensor 10 of the present embodiment, the reference electrode 21 is formed to be separated from the source electrode 15 and the drain electrode 16, but the arrangement of the reference electrode 21 is not limited thereto. The reference electrode 21 may be arranged on the same member as the source electrode 15 and the drain electrode 16.

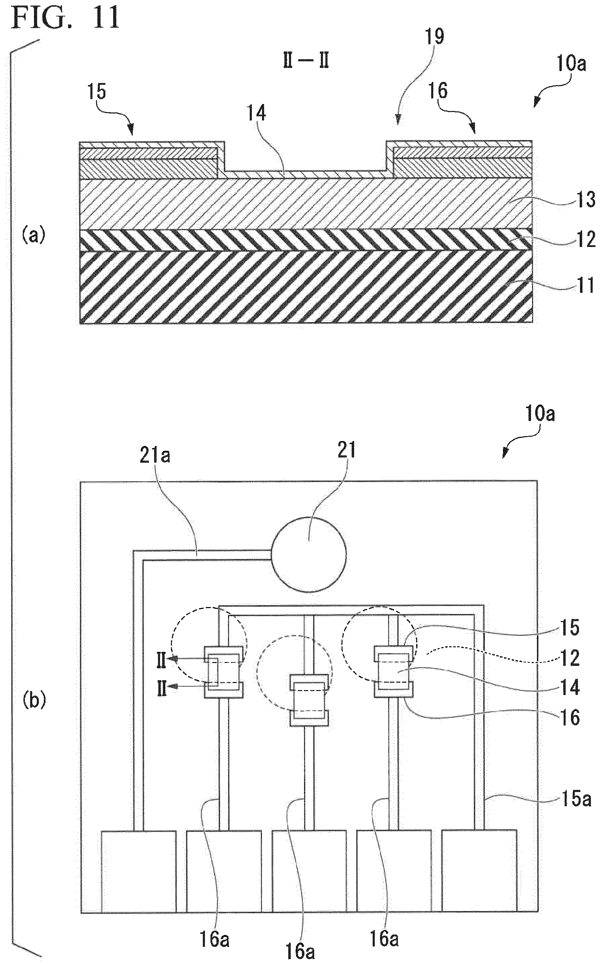

An example of the transistor sensor 10 in which the reference electrode 21 is arranged on the same member as the source electrode 15 and the drain electrode 16 on the substrate 11 is shown in Figs. 11. Fig. 11(a) is a cross-sectional view (cross-section along line II-II in Fig. 11(b)) of another example of the configuration of a transistor sensor according to the first embodiment of the present invention and Fig. 11(b) is a plan view thereof.

As shown in Fig. 11(a) and Fig. 11(b), a transistor sensor 10a has three of the back gate electrodes (conductive material layers) 12 provided on the substrate 11 and the gate insulating layer (solid electrolyte layer) 13 covering the back gate electrodes (conductive material layers) 12. The single reference electrode 21 is provided on the gate insulating layer (solid electrolyte layer) 13. In addition, three sensor pieces including the source electrode 15, the drain electrode 16, and the channel layer 14 are provided on the gate insulating layer (solid electrolyte layer) 13. Each of the three sensor pieces is arranged above the three back gate electrodes (conductive material layers) 12. In order to keep the distance from the reference electrode 21 to each sensor piece constant, the sensor pieces and the back gate electrodes (conductive material layer) 12 are arranged in concentric circles with the reference electrode 21 as the center. The reference electrode 21 is connected to a reference electrode drawer line 21a. The source electrode 15 of each sensor piece is connected to a single source electrode drawer line 15a. The drain electrode 16 of each sensor piece is connected to each separate drain electrode drawer line 16a.

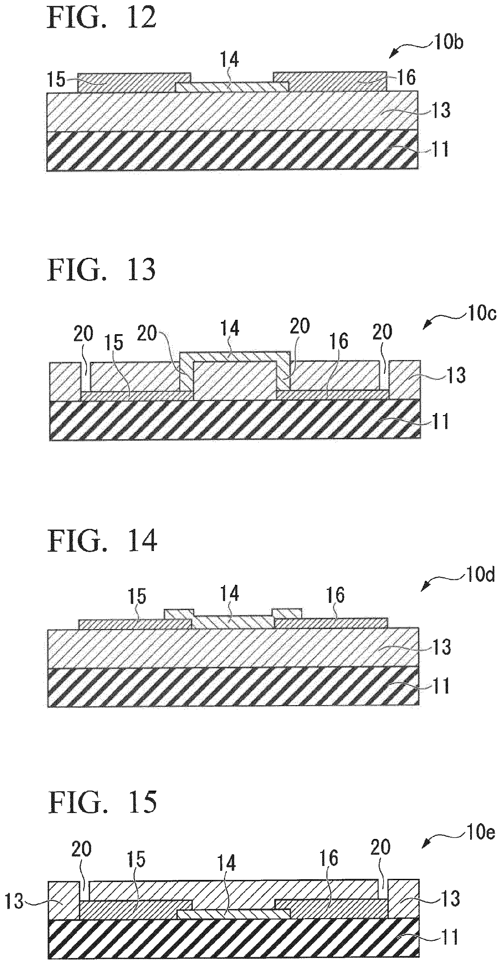

In addition, in the transistor sensor 10 of the present embodiment, the substrate 11, the back gate electrode (conductive material layer) 12, the gate insulating layer (solid electrolyte layer) 13, the channel layer 14, the source electrode 15, and the drain electrode 16 are provided in that order and the exposed portion 17 is formed to be at least a portion of the channel layer 14; however, the configuration is not limited thereto. Fig. 12 to Fig. 17 show yet more examples of the configuration of the transistor sensor according to the first embodiment of the present invention. Transistor sensors 10b to 10d shown in Fig. 12 to Fig. 14 have a configuration in which the exposed portion 17 is able to be at least a portion of the channel layer 14 or at least a portion of the channel layer 14 and the gate insulating layer (solid electrolyte layer) 13. Transistor sensors 10e to 10g shown in Fig. 15 to Fig. 17 have a configuration in which the exposed portion 17 is able to be at least a portion of the gate insulating layer (solid electrolyte layer) 13.

In the transistor sensor 10b shown in Fig. 12, the gate insulating layer (solid electrolyte layer) 13 is arranged on the substrate 11. The channel layer 14 is arranged on the gate insulating layer (solid electrolyte layer) 13 and the source electrode 15 and the drain electrode 16 are formed on the channel layer 14. The source electrode 15 and the drain electrode 16 are sized such that the edges thereof are inside the edges of the gate insulating layer (solid electrolyte layer) 13.

In the transistor sensor 10c shown in Fig. 13, the source electrode 15 and the drain electrode 16 are arranged on the substrate 11. The source electrode 15 and the drain electrode 16 are covered by the gate insulating layer (solid electrolyte layer) 13. The channel layer 14 is formed on the gate insulating layer (solid electrolyte layer) 13. The channel layer 14 is connected to the source electrode 15 and the drain electrode 16 through through holes 20 provided in the gate insulating layer (solid electrolyte layer) 13. In addition, the gate insulating layer (solid electrolyte layer) 13 is provided with the through holes 20 for connecting the source electrode 15 and the drain electrode 16 to an external power source.

In the transistor sensor 10d shown in Fig. 14, the gate insulating layer (solid electrolyte layer) 13 is arranged on the substrate 11. The source electrode 15 and the drain electrode 16 are arranged on the gate insulating layer (solid electrolyte layer) 13. The transistor sensor 10e shows a configuration of the transistor sensor 10 shown in Figs. 1 without being provided with the back gate electrode (conductive material layer) 12.

In the transistor sensor 10e shown in Fig. 15, the channel layer 14 is arranged on the substrate 11 and the source electrode 15 and the drain electrode 16 are formed on the channel layer 14. The channel layer 14, source electrode 15, and drain electrode 16 are covered by the gate insulating layer (solid electrolyte layer) 13. In the gate insulating layer (solid electrolyte layer) 13, the through holes 20 are provided for connecting the source electrode 15 and the drain electrode 16 to an external power source.

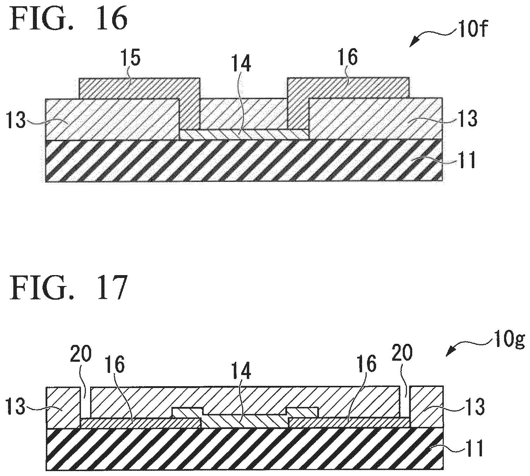

In the transistor sensor 10f shown in Fig. 16, the channel layer 14 is arranged on the substrate 11. The channel layer 14 is covered by the gate insulating layer (solid electrolyte layer) 13. The source electrode 15 and the drain electrode 16 are formed on the gate insulating layer (solid electrolyte layer) 13. The source electrode 15 and the drain electrode 16 are connected to the channel layer 14 through the through hole 20 provided in the gate insulating layer (solid electrolyte layer) 13.

In the transistor sensor 10g shown in Fig. 17, the source electrode 15 and the drain electrode 16 are arranged on the substrate 11 and the channel layer 14 is formed on the source electrode 15 and the drain electrode 16. The channel layer 14, source electrode 15, and drain electrode 16 are covered by the gate insulating layer (solid electrolyte layer) 13. In the gate insulating layer (solid electrolyte layer) 13, the through holes 20 are provided for connecting the source electrode 15 and the drain electrode 16 to an external power source.

In addition, in the transistor sensor 10 of the present embodiment, the back gate electrode (conductive material layer) 12 is arranged between the substrate 11 and the gate insulating layer (solid electrolyte layer) 13, but the arrangement of the back gate electrode (conductive material layer) 12 is not limited thereto. However, the back gate electrode (conductive material layer) 12 is preferably in contact with at least a portion of the gate insulating layer (solid electrolyte layer) 13. In addition, the back gate electrode (conductive material layer) 12 may be electrically insulated except at the gate insulating layer (solid electrolyte layer) 13. The back gate electrode (conductive material layer) 12 may, for example, be arranged inside the gate insulating layer (solid electrolyte layer) 13.

Furthermore, the transistor sensor 10 of the present embodiment may be a thin film transistor sensor in which the gate insulating layer (solid electrolyte layer) 13 of a solid electrolyte is formed between the substrate 11 or the back gate electrode (conductive material layer) 12 and the channel layer 14, in which the gate insulating layer (solid electrolyte layer) 13 is formed of any one of metal oxides including rare earth elements and zirconium (Zr) and metal oxides including rare earth elements and tantalum (Ta), the channel layer 14 is formed of a metal oxide including at least indium (In), the carbon (C) content ratio in the gate insulating layer (solid electrolyte layer) 13 is 0.5 atom% or more and 15 atom% or less, and the hydrogen (H) content ratio in the gate insulating layer (solid electrolyte layer) 13 is 2 atom% or more and 20 atom% or less.

### EXAMPLES

A description will be given below of Examples of the present invention. The present invention is not limited only to the following Examples.

### [Examples 1 and 2]

Using the manufacturing method described above, a transistor sensor was produced by forming a substrate, a gate electrode, a gate insulating layer (solid electrolyte layer), a channel layer, a source electrode, and a drain electrode in the shapes shown in Figs. 1 with the materials and thicknesses shown in Table 1. The material of the gate insulating layer (solid electrolyte layer) was LZO, the material of the channel layer was indium oxide (In₂O₃), and the width W and length L of the channel layer were set to 300 µm and 50 µm, respectively. In addition, the heating temperature when forming the gate insulating layer (solid electrolyte layer) was 400°C and the heating temperature when forming the channel layer was 300°C.

When the carbon (C) and hydrogen (H) content ratios in the gate insulating layer (solid electrolyte layer) were each measured using Rutherford Backscattering Spectrometry (RBS analysis method), Hydrogen Forward Scattering Spectrometry (HFS analysis method), and nuclear reaction analysis (NRA analysis method) (Pelletron 3 SDH manufactured by National Electrostatics Corporation), the carbon (C) moisture content ratio was 2.0 atom% and the hydrogen (H) content ratio was 10.1 atom%.

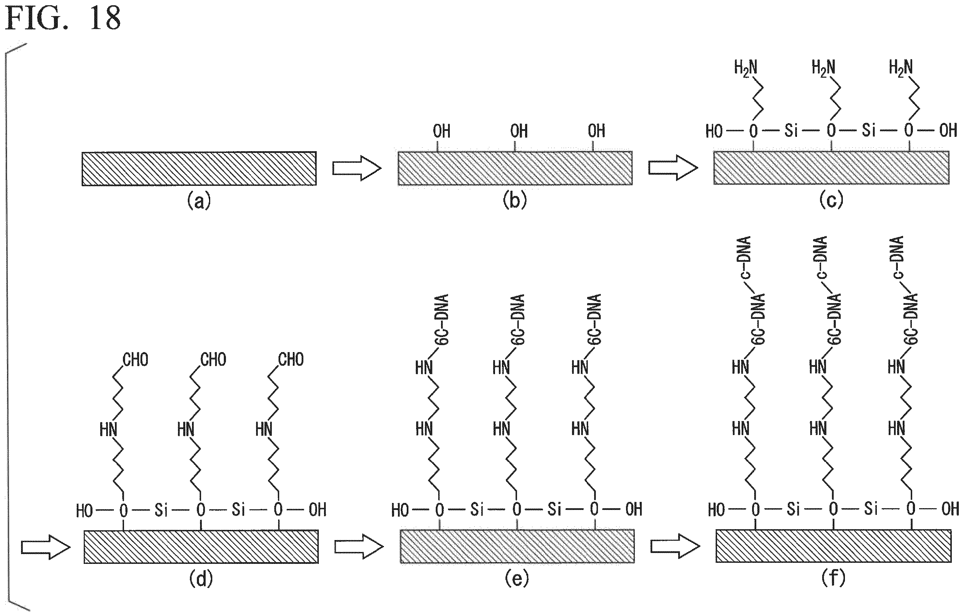

Next, after the channel layer was formed according to the manufacturing method described above (Fig. 18(a)), the surface of the channel layer was washed with ethanol and pure water. Next, a surface treatment was performed with a solution of hydrogen peroxide, ammonium water, and water mixed in a volume ratio (v/v) of 1:1:5 for 10 minutes at room temperature, followed by rinsing with pure water and drying the surface of the channel layer at 110°C for 10 minutes (Fig. 18(b)).

Next, a surface treatment was performed with an ethanol solution in which 3-aminopropyltriethoxysilane was mixed at 10% by volume in ethanol for 30 minutes at room temperature, followed by rinsing with pure water and drying at 120°C for 20 minutes (Fig. 18(c)). Then, a surface treatment was performed with a solution in which glutaraldehyde was dissolved in a phosphate buffer at 2.5% by volume for 30 minutes at room temperature and washing was carried out with pure water (Fig. 18(d)). Thereafter, a liquid including probe DNA was supplied to the surface of the channel layer and the probe DNA was fixed by chemical bonding (Fig. 18(e)). Due to this, a capturing field for indirectly capturing biomaterials, such as single-stranded target DNA, through the probe DNA was formed on the channel layer. As the probe DNA, a single-stranded probe DNA (also referred to as 6C-DNA) with a linker formed of an amino group and six carbons on the 5' side was used and it was confirmed to have the sequence shown by "20 bases; 6232 MW; Tm 57°C, 5'-[AmC6]CC TA TC GC TG CT AC CG TG AA-3‴.

Thereafter, target DNA (also referred to as cDNA), which forms complementary DNA with respect to the probe DNA, was supplied thereto and the target DNA was captured on the channel layer by hybridization of the probe DNA and the target DNA (Fig. 18(f)). It was confirmed that the target DNA had the sequence shown as "20 bases; 6182 MW; Tm 57°C, 5'-TT CA CG GT AG CA GC GA TA GG-3'".

### (Example 1)

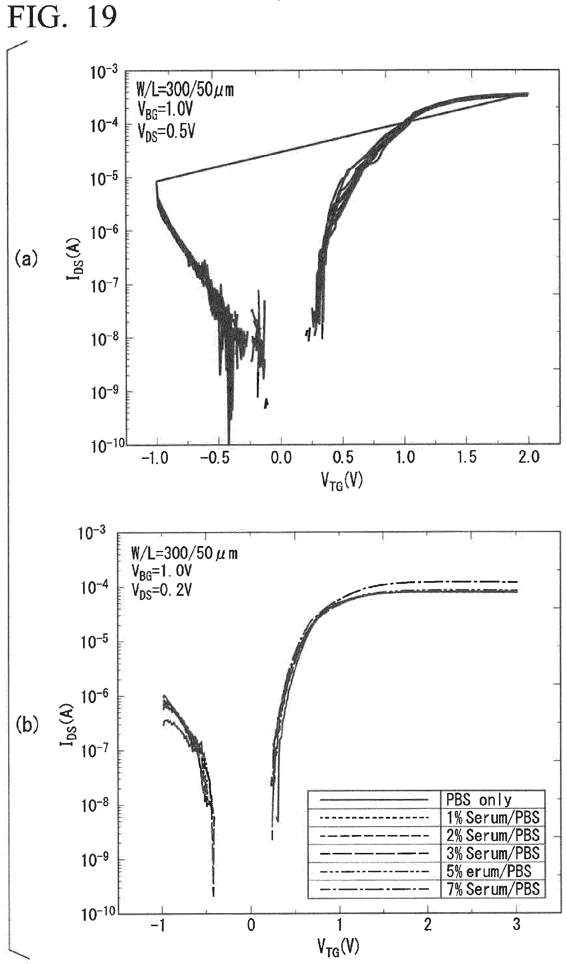

A phosphate buffer (PBS) was supplied on the channel layer, the voltage V_{BG} applied between the source electrode and the back gate electrode (conductive material layer) was set to 1.0V, the voltage V_{DS} applied between the source electrode and the drain electrode was set to 0.5V, and the V_{TG}-I_{DS} characteristics were measured. The results are shown in Fig. 19(a).

From the results in Fig. 19(a), even in a case of performing ten repeated measurements, almost no change was seen in the V_{TG}-I_{DS} characteristics and it was understood that the transistor sensor exhibited high detection stability in a phosphate buffer.

### (Example 2)

A plurality of mixed solutions with serum concentrations of 1%, 2%, 3%, 5%, and 7% with respect to a phosphate buffer, respectively, were supplied on the channel layer, the voltage V_{BG} was set to 1.0V, the voltage VDS was set to 0.2V, and the V_{TG}-I_{DS} characteristics were measured. The results are shown in Fig. 19(b).

From the results in Fig. 19(b), almost no change in the V_{TG}-I_{DS} characteristics was seen even when the concentration of serum in the mixed solution was changed in a range of 1 % to 7%. Thus, it was understood that the transistor sensor exhibited high detection stability even in a mixed solution including serum.

In the Examples, it is assumed that the reason why the transistor sensor exhibited high detection stability both in a phosphate buffer and in a mixed solution including serum is that the transistor sensor is extremely stable in an aqueous solution and effectively suppresses non-specific binding in the gate insulating layer (solid electrolyte layer) and/or channel layer.

### [Example 3]

### (Invention Example 1)

A transistor was produced under the conditions shown in Table 1 above. Specifically, the transistor was produced in the following manner.

A SiO₂/Si substrate formed of a silicon oxide (SiO₂) film with a thickness of 500 nm was prepared on a silicon substrate. A back gate electrode (conductive material layer) formed of a conductive material layer with a Pt/Ti two-layer structure was formed by depositing a titanium (Ti) layer with a thickness of 10 nm and a platinum (Pt) layer with a thickness of 100 nm on the silicon oxide film of the SiO₂/Si substrate in that order by a sputtering method.

A gate insulating layer (solid electrolyte layer) was formed by the sol-gel method on the obtained back gate electrode (conductive material layer). First, a precursor layer for the gate insulating layer (solid electrolyte layer) was formed by coating a La_{0.5}Zr_{0.5}O solution as a precursor solution for the gate insulating layer (solid electrolyte layer) by the spin coating method. Next, the precursor layer for the gate insulating layer (solid electrolyte layer) was pre-fired at 250°C in an oxygen-containing atmosphere, followed by main firing at 400°C to form a gate insulating layer (solid electrolyte layer) formed of an La_{0.5}Zr_{0.5}O layer (solid electrolyte layer) with a thickness of 120 µm. The La_{0.5}Zr_{0.5}O solution was prepared as follows.

Lanthanum acetate 1.5 hydrate and zirconium butoxide were mixed in a ratio of 1:1 (molar ratio) and the obtained mixture was dissolved in propionic acid to be 0.2 mol/kg in terms of the La_{0.5}Zr_{0.5}O concentration. The obtained mixed solution was refluxed in an oil bath at 110°C for 30 minutes and then filtered through a membrane filter with a pore size of 0.2 µm to obtain 0.2 mol/kg of an La_{0.5}Zr_{0.5}O solution.

A resist film patterned in the shape of the source electrode and drain electrode was formed on the obtained gate insulating layer (solid electrolyte layer) by the photolithography method. Next, an ITO layer with a thickness of 50 nm and a platinum (Pt) layer with a thickness of 100 nm were deposited in that order by the sputtering method on the gate insulating layer (solid electrolyte layer) on which the resist film was formed and the resist film was removed. The source electrode and drain electrode with a two-layered structure of Pt/ITO were formed. The size of each of the source electrode and the drain electrode was 320 µm wide x 200 µm long and the distance between the source electrode and drain electrode was 50 µm.

Next, a resist film patterned in the shape of the channel layer was formed on the gate insulating layer (solid electrolyte layer) by the photolithography method. Next, an In₂O₃ solution as a precursor solution for the channel layer was coated by the spin coating method on the gate insulating layer (solid electrolyte layer) on which the resist film was formed to form a precursor layer for the channel layer. Next, the channel layer formed of In₂O₃ (inorganic semiconductor) with a thickness of 20 µm was formed by pre-firing the precursor layer for the channel layer at 250°C and then carrying out main firing at 300°C in an oxygen-containing atmosphere. The size of the channel layer was 300 µm wide x 50 µm long. The In₂O₃ solution was prepared as follows.

Indium nitrate trihydrate was dissolved in 2-methoxyethanol to be 0.2 mol/kg in terms of the In₂O₃ concentration. The obtained solution was refluxed in an oil bath at 110°C for 30 minutes and then filtered through a membrane filter with a pore size of 0.2 µm to obtain an In₂O₃ solution with a concentration of 0.2 mol/kg.

### (TFT Sensor of Related Art)

A TFT sensor of the related art was produced in the same manner as in Invention Example 1, except that the temperature of the main firing of the precursor layer for the gate insulating layer (solid electrolyte layer) was set to 550°C and the temperature of the main firing of the precursor layer for the channel layer was set to 500.

### (Invention Example 2)

The transistor sensor of Invention Example 2 was produced in the same manner as in Invention Example 1 except that an Sm_{0.1}Zr_{0.9}O solution was used as the precursor solution for the gate insulating layer (solid electrolyte layer) to form a gate insulating layer (solid electrolyte layer) formed of an Sm_{0.1}Zr_{0.9}O layer. The Sm_{0.1}Zr_{0.9}O solution was prepared as follows.

Samarium acetate tetrahydrate and zirconium butoxide were mixed in a ratio of 1:9 (molar ratio) and the obtained mixture was dissolved in propionic acid to be 0.2 mol/kg in terms of the Sm_{0.1}Zr_{0.9}O concentration. The obtained mixed solution was refluxed in an oil bath at 110°C for 30 min and then filtered through a membrane filter with a pore size of 0.2 µm to obtain an Sm_{0.1}Zr_{0.9}O solution with a concentration of 0.2 mol/kg.

### (Invention Example 3)

The transistor sensor of Invention Example 3 was produced in the same manner as in Invention Example 1 except that an SM_{0.3}Zr_{0.7}O solution was used as the precursor solution for the gate insulating layer (solid electrolyte layer) to form a gate insulating layer (solid electrolyte layer) formed of an Sm_{0.3}Zr_{0.7}O layer. The Sm_{0.3}Zr_{0.7}O solution was prepared in the same manner as in Invention Example 3, except that samarium acetate tetrahydrate and zirconium butoxide were mixed in a ratio of 3:7 (molar ratio).

### (Invention Example 4)

The transistor sensor of Invention Example 4 was produced in the same manner as in Invention Example 1 except that a Dy_{0.1}Zr_{0.9}O solution was used as the precursor solution for the gate insulating layer (solid electrolyte layer) to form a gate insulating layer (solid electrolyte layer) formed of a Dy_{0.1}Zr_{0.9}O layer. The Sm_{0.1}Zr_{0.9}O solution was prepared as follows.

Dysprosium acetate tetrahydrate and zirconium butoxide were mixed in a ratio of 1:9 (molar ratio) and the obtained mixture was dissolved in propionic acid to be 0.2 mol/kg in terms of the Dy_{0.1}Zr_{0.9}O concentration. The obtained mixed solution was refluxed in an oil bath at 110°C for 30 min and then filtered through a membrane filter with a pore size of 0.2 µm to obtain a Dy_{0.1}Zr_{0.9}O solution with a concentration of 0.2 mol/kg.

### (Invention Example 5)

The transistor sensor of Invention Example 5 was produced in the same manner as in Invention Example 1 except that a Dy_{0.3}Zr_{0.7}O solution was used as the precursor solution for the gate insulating layer (solid electrolyte layer) to form a gate insulating layer (solid electrolyte layer) formed of a Dy_{0.3}Zr_{0.7}O layer. The Dy_{0.3}Zr_{0.7}O solution was prepared in the same manner as in Example 5, except that dysprosium acetate tetrahydrate and zirconium butoxide were mixed in a ratio of 3:7 (molar ratio).

### (Invention Example 6)

The transistor sensor of Invention Example 6 was produced in the same manner as in Invention Example 1 except that a La_{0.3}Ta_{0.7}O solution was used as the precursor solution for the gate insulating layer (solid electrolyte layer) to form a gate insulating layer (solid electrolyte layer) formed of an La_{0.3}Ta_{0.7}O layer. The La_{0.3}Ta_{0.7}O solution was prepared as follows.

Lanthanum acetate 1.5 hydrate and tantalum butoxide were mixed in a ratio of 3:7 (molar ratio) and the obtained mixture was dissolved in propionic acid to be 0.2 mol/kg in terms of the La_{0.3}Ta_{0.7}O concentration. The obtained mixed solution was refluxed in an oil bath at 110°C for 30 min and then filtered through a membrane filter with a pore size of 0.2 µm to obtain an La_{0.3}Ta_{0.7}O solution with a concentration of 0.2 mol/kg.

### (Invention Example 7)

The transistor sensor of Invention Example 7 was produced in the same manner as in Invention Example 1 except that a La_{0.3}Ta_{0.7}O solution was used as the precursor solution for the gate insulating layer (solid electrolyte layer) to form a gate insulating layer (solid electrolyte layer) formed of an La_{0.3}Ta_{0.7}O layer, a ZnO solution was used as the precursor solution for the channel layer to form a channel layer formed of a ZnO layer, and the size of the channel layer was 50 µm wide × 10 µm long. The La_{0.3}Ta_{0.7}O solution was prepared in the same manner as in Invention Example 1, except that lanthanum acetate 1.5 hydrate and zirconium butoxide were mixed in a ratio of 3:7 (molar ratio).

In addition, the ZnO solution was prepared as follows.

Zinc acetate dihydrate, acetylacetone, and ammonium acetate were mixed at a ratio of 1:1:1 (molar ratio), respectively, and the obtained mixture was dissolved in 2-methoxyethanol to be 0.4 mol/kg in terms of the ZnO concentration. The obtained solution was refluxed in an oil bath at 110°C for 30 minutes and then filtered through a membrane filter with a pore size of 0.2 µm to obtain a ZnO solution with a concentration of 0.4 mol/kg.

### (Invention Example 8)

The transistor sensor of Invention Example 8 was produced in the same manner as in Invention Example 7 except that the size of the channel layer was set to 300 µm wide x 50 µm long.

### (Invention Example 9)

The transistor sensor of Invention Example 10 was produced in the same manner as in Invention Example 7 except that an In_{0.33}Ga_{0.33}Zn_{0.33}O solution was used as the precursor solution for the channel layer to form a channel layer formed of an In_{0.33}Ga_{0.33}Zn_{0.33}O layer. The In_{0.33}Ga_{0.33}Zn_{0.33}O solution was prepared as follows.

Indium nitrate trihydrate, gallium nitrate n-hydrate (n = 7 to 9), and zinc acetate dihydrate were mixed at a ratio of 1:1:1 (molar ratio), respectively, and the obtained mixture was dissolved in 2-methoxyethanol to be 0.3 mol/kg in terms of the In_{0.33}Ga_{0.33}Zn_{0.33}O concentration. The obtained solution was refluxed in an oil bath at 110°C for 30 minutes and then filtered through a membrane filter with a pore size of 0.2 µm to obtain an In_{0.33}Ga_{0.33}Zn_{0.33}O solution with a concentration of 0.4 mol/kg.

### (Invention Example 10)

The transistor sensor of Invention Example 10 was produced in the same manner as in Invention Example 9 except that the size of the channel layer was set to 300 µm wide x 50 µm long.

### (Comparative Example 1)

The transistor sensor of Comparative Example 1 was produced in the same manner as in Invention Example 1 except that the back gate electrode (conductive material layer) and the gate insulating layer (solid electrolyte layer) were not provided, that is, the source electrode, the drain electrode, and the channel layer were formed on the silicon oxide film of the SiO₂/Si substrate.

### (Evaluation)

For the transistor sensors produced in Invention Examples 2 to 10, the carbon (C) and hydrogen (H) content ratios in the gate insulating layer (solid electrolyte layer) were measured by the method described above. For the transistor sensors produced in Invention Examples 2 to 10 and Comparative Example 1, the sensing characteristics were measured by the method described below. The results are shown in Table 2 below.

### (Method for Measuring Sensing Characteristics)

A square shaped DNA probe loading area (loading portion) of 750 µm x 750 µm was formed by forming a resist film using the photolithography method, centered on the channel layer. The DNA probe loading area (loading portion) includes the gate insulating layer (solid electrolyte layer) and the channel layer. Furthermore, a liquid holding portion including the channel layer and gate insulating layer (solid electrolyte layer) was formed by providing a wall portion around the periphery of the DNA probe loading area (loading portion). Next, probe DNA for capturing biomaterials which were the measurement target was loaded on the DNA probe loading area (loading portion) by the method described above.

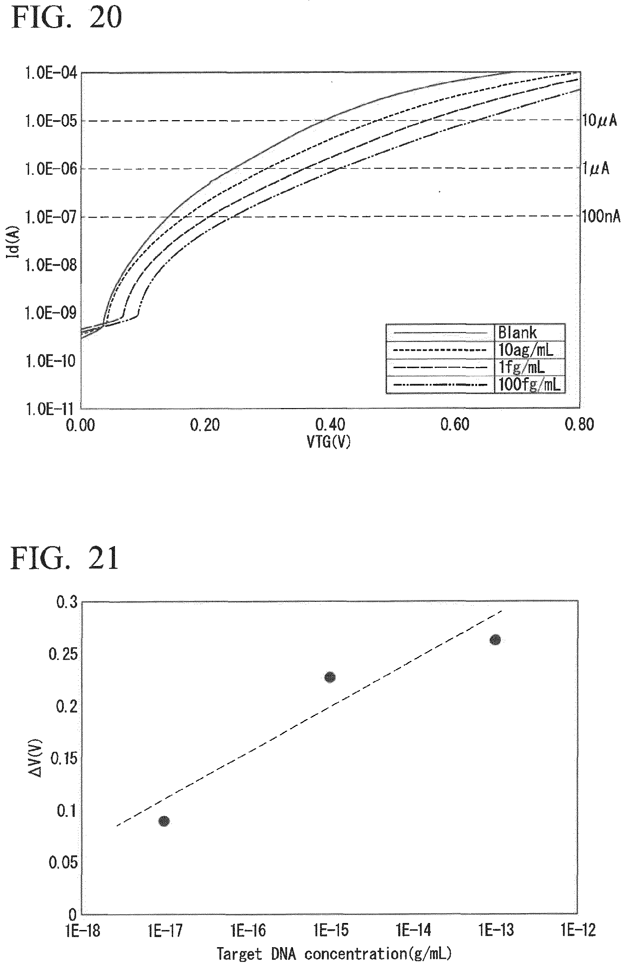

Target DNA solutions with target DNA concentrations of 10 ag/mL, 1 fg/mL, and 100 fg/mL and blanks were prepared as samples. Each sample was supplied to the liquid holding portion and the V_{TG}-I_{DS} characteristics thereof were measured. The measurement of V_{TG}-I_{DS} characteristics was performed under the condition of the voltage VDS applied between the source electrode and drain electrode being 0.2V An example of the V_{TG}-I_{DS} characteristics obtained from this measurement is shown in Fig. 20. From the graph in Fig. 20, the value of V_{TG} when I_{DS} reached 1 µA was read. A graph in which the target DNA concentration of the sample is plotted on the horizontal axis and the read V_{TG} value is plotted on the vertical axis is shown in Fig. 21. The slope of the line connecting the plotted points shown in Fig. 21 was set as the sensing characteristic (unit: mV/digit). A large value for the sensing characteristic indicates a high sensitivity. From the viewpoint of improving the sensitivity to target DNA and the detection stability by widening the detectable range of target DNA, the sensing characteristic is preferably 10 mV/decade or higher and particularly preferably 20 mV/decade or higher.

**[Table 2]**

| | Back gate electrode (conductive material layer) | | Gate insulating layer (solid electrolyte layer) | | | | | Channel layer | | | Size of DNA probe loading area (loading portion) | Sensing characteristi c (mV/decade ) |
|---|---|---|---|---|---|---|---|---|---|---|---|---|
| | Material | Thicknes s (Pt/Ti) | Materia 1 | Elemen t ratio (x:y) | Thicknes s | Carbon (C) content ratio | Hydroge n (H) content ratio | Material | Thicknes s | Size | | |
| Invention Example 2 | Pt/Ti | 100nm/ 10 nm | SmₓZr_{y} O | 0.1:0.9 | 120nm | 2.1atom % | 8.6atom% | In₂O₃ | 20nm | 300µm×50µ m | 750µm×750µ m | 13 |
| Invention Example 3 | Pt/Ti | 100nm/10 nm | SmₓZr_{y} O | 0.3:0.7 | 120nm | 2.6atom % | % 7.9atom% | In₂O₃ | 20nm | 300µm×50µ m | 750µm×750µ m | 15 |
| Invention Example 4 | Pt/Ti | 100nm/10 nm | DyₓZr_{y} O | 0.1:0.9 | 120nm | 3.5atom % | 11.2atom % | In₂O₃ In₂O₃ | 20nm | 300wm×50µ m | 750µm×750µ m | 17 |
| Invention Example 5 | Pt/Ti | 100nm/10 nm | DyₓZr_{y} O | 0.3:0.7 | 120nm | 4.6atom % | 10.5atom % | In₂O₃ | 20nm | 300µm×50µ m | 750µm×750µ m | 19 |
| Invention Example 6 | Pt/Ti | 100nm/10 nm | LaₓTa_{y}O | 0.3:0.7 | 120nm | 0.4atom % | % 0.8atom% | In₂O₃ | 20nm | 300µm×50µ m | 750µm×750µ m | 10 |
| Invention Example 7 | Pt/Ti | 100nm/10 nm | LaₓZr_{y}O | 0.3:0.7 | 120nm | 1.8atom% % | 14.6atom% % | ZnO | 20nm | 50µm×10µm | 750µm×750µ m | 13 |
| Invention Example 8 | Pt/Ti | 100nm/10 nm | LaₓZr_{y}O | 0.3:0.7 | 120nm | 1.9atom % | 12.5atom % | ZnO | 20nm | 300µm×50µ m | 750µm×750µ m | 14 |
| Invention Example 9 | Pt/Ti | 100nm/10 nm | LaₓZr_{y}O | 0.3:0.7 | 120nm | 2.6atom % | 10.6atom % | In_{0.33}Ga_{0.33}Zn_{0.33} O | 20nm | 50nm×10um | 750µm×750µ m | 11 |
| Invention Example 10 | Pt/Ti | 100nm/10 nm | LaₓZr_{y}O | 0.3:0.7 | 120nm | 2.9atom% % | 10.7atom% % | In_{0.33}Ga_{0.33}Zn_{0.33} O | 20nm | 300µm×50µ m | 750µm×750µ m | 11 |
| Comparativ e Example 1 | Pt/Ti | 100nm/10 nm | - | - | - | - | - | In₂O₃ | 20nm | 300µm×50µ m | 750µm×750µ m | 2 |

From the results shown in Table 2, it is understood that the transistor sensors of Invention Examples 2 to 10, in which the gate electrode layer is a solid electrolyte layer including an inorganic solid electrolyte, have a sensing characteristic of 10 mV/decade or more, which improves the sensitivity with respect to target DNA and the detection stability.

### (Invention Examples 11 to 14)

The transistor sensors of Invention Examples 11 to 14 were produced in the same manner as in Invention Example 1 except that an La_{0.3}Zr_{0.7}O solution was used as a precursor solution for the gate insulating layer (solid electrolyte layer) to form a gate insulating layer (solid electrolyte layer) formed of an La_{0.3}Zr_{0.7}O layer. The La_{0.3}Zr_{0.7}O solution was prepared as follows.

Lanthanum acetate 1.5 hydrate and zirconium butoxide were mixed in a ratio of 3:7 (molar ratio) and the obtained mixture was dissolved in propionic acid to be 0.2 mol/kg in terms of the La_{0.3}Zr_{0.7}O concentration. The obtained mixed solution was refluxed in an oil bath at 110°C for 30 minutes and then filtered through a membrane filter with a pore size of 0.2 µm to obtain an La_{0.3}Zr_{0.7}O solution with a concentration of 0.2 mol/kg.

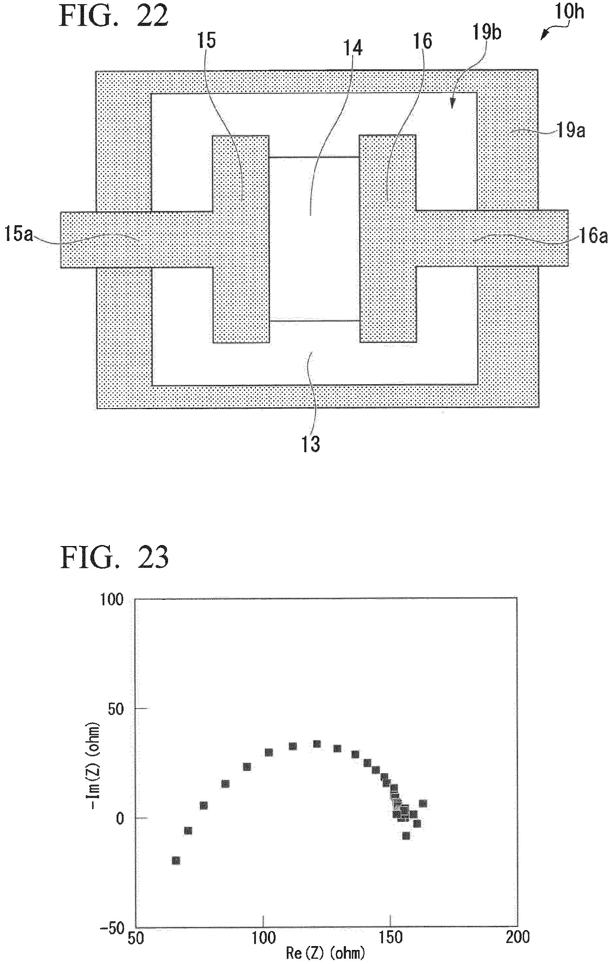

In Invention Examples 11 to 14, the sensing characteristics of the obtained transistor sensors were measured by changing the size of the DNA probe loading area (loading portion) for capturing the target DNA by loading probe DNA, that is, the size of the portion in contact with the target DNA solution. The size of the DNA probe loading area was adjusted by forming a resist film on the surface of the transistor sensor using the photolithography method. Fig. 22 is a plan view of the transistor sensor produced in Invention Example 12. As shown in Fig. 22, a transistor sensor 10h has the channel layer 14, and the source electrode 15 and the drain electrode 16 connected to the channel layer 14. The source electrode 15 is connected to the source electrode drawer line 15a and the drain electrode 16 is connected to the drain electrode drawer line 16a. The periphery of the channel layer 14, the source electrode 15 and the source electrode drawer line 15a, and the drain electrode 16 and the drain electrode drawer line 16a are covered with a resist film 19a and the portion not covered with the resist film 19a is a DNA probe loading area 19b, that is, the portion in contact with the target DNA solution. The size of the channel layer 14 is 300 µm × 50 µm and the size of the DNA probe loading area 19b is 500 µm × 500 µm. In Invention Example 11, the size of the DNA probe loading area 19b was 300 µm × 50 µm and, other than the channel layer 14, was covered with the resist film 19a. In Invention Example 13, the size of the DNA probe loading area 19b was 750 µm × 750 µm. In Invention Example 14, the covering with the resist film 19a was not carried out.

**[Table 3]**

| | Back gate electrode (conductive material layer) | | Gate insulating layer (solid electrolyte layer) | | | | Channel layer | | | Size of DNA probe loading area (loading portion) | Sensing characteristic (mV/decade) |
|---|---|---|---|---|---|---|---|---|---|---|---|
| | Material | Thickness (Pt/Ti) | Material | Thickness | Carbon (C) content ratio | Hydrogen (H) content ratio | Material | Thickness | Size | | |
| Invention Example 11 | Pt/Ti | 100nm/10nm | La_{0.3}Zr_{0.7}O | 120nm | 2.2atom% | 11.1atom% | In₂O₃ | 20nm | 300µm×50µm | 300µm×50µm | 16 |
| Invention Example 12 | Pt/Ti | 100nm/10nm | La_{0.3}Zr_{0.7}O | 120nm | 2.3atom% | 13.1atom% | In₂O₃ | 20nm | 300µm×50µm | 500µm×500µm | 29 |
| Invention Example 13 | Pt/Ti | 100nm/10nm | La_{0.3}Zr_{0.7}O | 120nm | 2.1atom% | 12.1atom% | In₂O₃ | 20nm | 300µm×50µm | 750µm×750µm | 43 |
| Invention Example 14 | Pt/Ti | 100nm/10nm | La_{0.3}Zr_{0.7}O | 120nm | 2.2atom% | 10.9atom% | In₂O₃ | 20nm | 300µm×50µm | None | 54 |

From the results shown in Table 3, it is understood that the sensing characteristics improve along with the increase in the area of the DNA probe loading area 19b (loading area). This is considered to be due to the fact that the electric field created by the charge of the target DNA captured in the probe DNA in the gate insulating layer (solid electrolyte layer) is transmitted to the channel layer, which increases the effect (antenna effect) of increasing the amount of change in the electrical characteristics.

### (Invention Example 14)

By performing AC impedance measurements as follows, it was confirmed that the metal oxide (RZrO) including rare earth elements (R) and zirconium (Zr) used in the present Example is a solid electrolyte film, that is, exhibits ionic conductivity.

The sample for measurement was produced as follows. First, a Ti/Pt electrode film (lower electrode film) was formed on a SiO₂/Si substrate by the sputtering method in the same manner as in Invention Example 1. Next, an RZrO film with a thickness of 120 nm was formed on the lower electrode film by the sol-gel method. The SiO₂/Si substrate with the attached RZrO film was cut out into a square of 20 × 20 mm² and a square ITO/Pt electrode film (upper electrode film) with an area of 5 × 5 mm² was formed by the sputtering method by arranging a stainless-steel contact mask with a square hole of 5 × 5 mm² in the center on the RZrO film. In this manner, a sample for measurement was prepared in which the SiO₂/Si substrate, lower electrode film, RZrO film, and upper electrode film were laminated in this order.

The measurements were performed using an AC impedance measurement apparatus (manufactured by BioLogic, SP-300). The lower electrode film and upper electrode film of the sample for measurement described above and the AC impedance measurement apparatus were connected using Al tab electrode tape and the lower electrode film and upper electrode film and the Al tab electrode tape were bonded using Ag paste. The measurement conditions were AC frequency range: 0.1 to 7 MHz and AC voltage amplitude: 0.1 mV. A graph of the Cole-Cole plots of the real and imaginary parts of the AC impedance measured in the sample where the RZrO film is a LaZrO film including the rare earths La and Zr (LaZrO, La:Zr=3:7) is shown in Fig. 23. As shown in Fig. 23, an arc appears in the profile and, from analysis of the arc, the conductivity was 6.0 × 10⁻⁷ S/cm. The ionic conductivity was measured in the same manner for the other RZrO films and it was confirmed that the RZrO films used in the present Invention Examples all had a value of 1.0×10⁻⁸S/cm or more.

### [Reference Signs List]

10, 10a, 10b, 10c, 10e, 10f, 10g, 10h: Transistor sensor
11: Substrate
12: Back gate electrode (conductive material layer)
13: Gate insulating layer (solid electrolyte layer)
14: Channel layer
14a: Surface
15: Source electrode
15a: Source electrode drawer line
16: Drain electrode
16a: Drain electrode drawer line
17: Exposed portion
18: Liquid holding portion
19a: Resist film
19b: DNA probe loading area
20: Through hole
21: Reference electrode
21a: Reference electrode drawer line
22: Wall portion
23: First voltage supply portion
24: Second voltage supply portion
31: Probe DNA
32: Target DNA

## Claims

1. A transistor sensor comprising:
a substrate;
a channel layer provided over one surface of the substrate; and
a solid electrolyte layer provided between the substrate and the channel layer or over a surface of the channel layer on an opposite side to the substrate side,
wherein the channel layer includes an inorganic semiconductor,
the solid electrolyte layer includes an inorganic solid electrolyte, and
at least a portion of either one or both of the channel layer and the solid electrolyte layer includes an exposed portion exposed to outside.

2. The transistor sensor according to claim 1,
wherein the solid electrolyte layer is formed of any of a metal oxide including rare earth elements and zirconium (Zr) and a metal oxide including rare earth elements and tantalum (Ta),
the channel layer is formed of a metal oxide including at least indium (In),
a carbon (C) content ratio in the solid electrolyte layer is 0.5 atom% or more and 15 atom% or less, and
a hydrogen (H) content ratio in the solid electrolyte layer is 2 atom% or more and 20 atom% or less.

3. The transistor sensor according to claim 1 or 2,
wherein the solid electrolyte layer has an ionic conductivity of 1 × 10⁻⁸ S/cm or higher.

4. The transistor sensor according to any one of claims 1 to 3,
wherein a surface of the exposed portion has a capturing field configured to capture a biomaterial directly or indirectly.

5. The transistor sensor according to claim 4,
wherein a probe molecule for capturing the biomaterial is fixed to the capturing field.

6. The transistor sensor according to any one of claims 1 to 5, further comprising: a holding portion configured to hold a liquid including a biomaterial, in an upper portion of the exposed portion.

7. The transistor sensor according to any one of claims 1 to 6, further comprising:
a conductive material layer in contact with at least a portion of the solid electrolyte layer,
wherein the conductive material layer is electrically insulated except at the solid electrolyte layer.

8. The transistor sensor according to any one of claims 1 to 7, further comprising:
a source electrode and a drain electrode connected to the channel layer;
a reference electrode which, when a liquid including a biomaterial is arranged in contact with the exposed portion, is inserted into the liquid including the biomaterial; and
a detection portion configured to detect the biomaterial based on a voltage between the reference electrode and the source electrode and a current between the source electrode and the drain electrode.

9. A method for detecting a biomaterial using the transistor sensor according to claim 8, the method comprising:
a step of supplying a liquid including a biomaterial to the exposed portion;
a step of inserting the reference electrode into the liquid including the biomaterial; and
a step of applying a voltage between the reference electrode and the source electrode of the transistor sensor, measuring a current between the source electrode and the drain electrode of the transistor sensor, and detecting the biomaterial based on the voltage between the reference electrode and the source electrode and the current between the source electrode and the drain electrode.
